# EUROPÄISCHE PATENTSCHRIFT

(11) **EP 0 906 911 B1**
(45) Veröffentlichungstag und Bekanntmachung des Hinweises auf die Patenterteilung: **28.04.2004**
(21) Anmeldenummer: 98117810.6
(22) Anmeldetag: 19.09.1998
(51) Int. Cl.: C07D 311/68, A61K 31/35

(54) **Sulfonamid-substituierte Chromane (K+Kanal-Blocker), Verfahren zu ihrer Herstellung, ihre Verwendung als Medikament sowie sie enthaltende pharmazeutische Zubereitungen**
Sulfonamid-substituted chromane derivatives (K+channel-blocker), process for their preparation, their use as drugsand pharmaceutical compositions containing them
Dérivés sulfonamide de chromane substitués ayant une activité de bloquage de canaux de potassium, procédé pour leur préparation, utilisation comme médicaments et compositions pharmaceutiques les contenant

(30) Priorität: 26.09.1997 DE 19742508
(43) Veröffentlichungstag der Anmeldung: 07.04.1999
(73) Patentinhaber: Aventis Pharma Deutschland GmbH, 65929 Frankfurt am Main (DE)
(72) Erfinder: Brendel, Joachim Dr., 61118 Bad Vilbel (DE); Gerlach, Uwe Dr., 65795 Hattersheim (DE); Lang, Hans Jochen Dr., 65719 Hofheim (DE); Weidmann, Klaus Dr., 61476 Kronberg (DE)

(56) Entgegenhaltungen:
- EP-A- 0 370 901
- EP-A- 0 389 861
- EP-A- 0 807 629
- EP-A- 0 860 440
- BUSCH A E ET AL: "Role of the ISK protein in the IminK channel complex" TRENDS IN PHARMACOLOGICAL SCIENCES, Bd. 18, Nr. 1, Januar 1997, Seite 26-29 XP004049948
- LOHRMANN E ET AL: "A NEW CLASS OF INHIBITORS OF CAMP-MEDIATED CL- SECRETION IN RABBIT COLON, ACTING BY THE REDUCTION OF CAMP-ACTIVATED K+ CONDUCTANCE" PFLUEGERS ARCHIV, Bd. 429, 1995, Seiten 517-530, XP002038768
- SUESSBRICH,H. ET AL.: "Specific blockade of slowly activating IsK channels by chromanols - impact on the role of IsK channels in epithelia" FEBS LETTERS, Bd. 396, 1996, Seiten 271--275, XP002088856 AMSTERDAM
- SOLL,R.M. ET AL.: "N-Sulfonamides of Benzopyran-Related Potassium Openers: Conversion of Glyburide Insensitive Smooth Muscle Relaxants to Potent Smooth Muslce Contractors " BIOORG.&MED.CHEM.LETT., Bd. 4, Nr. 5, 1994, Seiten 769-773, XP002088855 OXFORD
- BUSCH,A.E. ET AL.: "The novel class III antiarrhythmics NE-10064 and NE-10133 inhibit Isk channels expressed in xenopus oocytes and Iks in guinea pig cardiac myocytes " BIOCHIM.BIOPHYS.ACTA, Bd. 202, Nr. 1, 1994, Seiten 265-270, XP002088857
- COLATSKY T J ET AL: "POTASSIUM CHANNEL BLOCKERS AS ANTIARRHYTHMIC DRUGS" DRUG DEVELOPMENT RESEARCH, Bd. 33, Nr. 3, 1. November 1994, Seiten 235-249, XP000567177

## Beschreibung

Die Erfindung betrifft Verbindungen der Formel I, 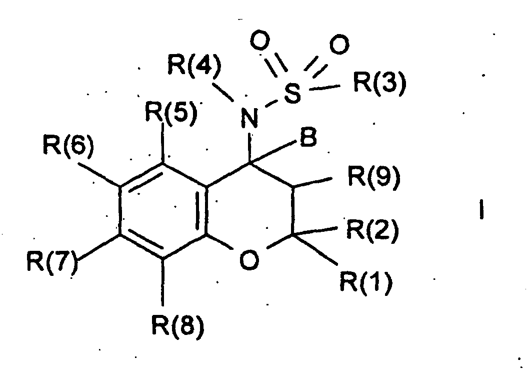 worin R(1), R(2), R(3), R(4), R(5), R(6), R(7), R(8), R(9) und B die im folgenden angegebenen Bedeutungen haben, ihre Herstellung und ihre Verwendung, insbesondere in Arzneimitteln. Die Verbindungen beeinflussen den durch cyclisches Adenosinmonophosphat (cAMP) geöffneten Kaliumkanal bzw. den I_{Ks}-Kanal und eignen sich in hervorragender Weise als Arzneimittelwirkstoffe, beispielsweise zur Prophylaxe und Therapie von Herz-Kreislauferkrankungen, insbesondere Arrhythmien, zur Behandlung von Ulcera des Magen-Darm-Bereichs oder zur Behandlung von Durchfallerkrankungen.

In der Arzneimittelchemie wurde in den letzten Jahren die Klasse der 4-Acylaminochroman-Derivate intensiv bearbeitet. Prominentester Vertreter dieser Klasse ist das Cromakalim der Formel A (J. Med. Chem. 1986, 29, 2194).

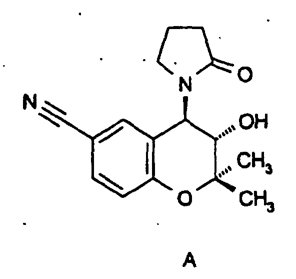

Bei Cromakalim und anderen verwandten 4-Acylaminochroman-Derivaten handelt es sich um Verbindungen mit relaxierender Wirkung auf glattmuskuläre Organe, so daß sie zur Senkung des erhöhten Blutdruckes infolge Gefäßmuskelrelaxation und in der Behandlung des Asthmas infolge der Relaxation der glatten Muskulatur der Atemwege verwendet werden. All diesen Präparaten ist gemeinsam, daß sie auf zellulärer Ebene beispielsweise von glatten Muskelzellen wirken und dort zu einer Öffnung bestimmter ATP-sensitiver K⁺-Kanäle führen. Der durch den Austritt von K⁺-Ionen induzierte Anstieg von negativer Ladung in der Zelle (Hyperpolarisation) wirkt über Sekundärmechanismen der Erhöhung der intrazellulären Ca²⁺-Konzentration und damit einer Zellaktivierung entgegen, die z. B. zu einer Muskelkontraktion führt.

Von diesen Acylamino-Derivaten unterscheiden sich die erfindungsgemäßen Verbindungen der Formel I strukturell unter anderem durch den Ersatz der Acylaminogruppe durch eine Sulfonylaminofunktion. Während Cromakalim (Formel A) und analoge Acylaminoverbindungen als Öffner ATP-sensitiver K⁺-Kanäfe wirken, zeigen die erfindungsgemäßen Verbindungen der Formel I mit der Sulfonylaminostruktur jedoch keine öffnende Wirkung auf diesen K⁺(ATP)-Kanal, sondern überraschenderweise eine starke und spezifische blockierende (schließende) Wirkung auf einen K⁺-Kanal, der durch cyclisches Adenosinmonophosphat (cAMP) geöffnet wird und sich grundlegend vom erwähnten K⁺(ATP)-Kanal unterscheidet. Neuere Untersuchungen zeigen, daß dieser an Dickdarmgewebe identifizierte K⁺ (cAMP) -Kanal sehr ähnlich, vielleicht sogar identisch, mit dem am Herzmuskel identifizierten I_{Ks}-Kanal ist. In der Tat konnte für die erfindungsgemäßen Verbindungen der Formel I eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP) -Kanals bzw. des I_{Ks}-Kanals entwickeln die erfindungsgemäßen Verbindungen im lebenden Organismus pharmakologische Wirkungen von hoher therapeutischer Verwertbarkeit.

Neben den oben genannten Cromakalim- bzw. Acylaminochroman-Derivaten werden in der Literatur auch Verbindungen mit 4-Sulfonylaminochroman-Struktur beschrieben, die sich aber sowohl in der Struktur als auch in der biologischen Wirkung deutlich von den erfindungsgemäßen Verbindungen der Formel I unterscheiden. So werden in der EP-A-315 009 Chromanderivate mit 4-Phenylsulfonylaminostruktur beschrieben, die sich durch antithrombotische und antiallergische Eigenschaften auszeichnen. In der EP-A-389 861 und der JP 01294677 werden 3-Hydroxychroman- bzw. Chromen-Derivate mit einer cyclischen 4-Sulfonylaminogruppe beschrieben (z.B. Verbindung B), die über eine Aktivierung des K⁺(ATP)-Kanals als Antihypertensiva wirken sollen. In der EP-A-370 901 werden 3-Hydroxychroman- bzw. Chromen-Derivate mit einer 4-Sulfonylaminogruppe, wobei die restliche Valenz des N-Atoms ein Wasserstoffatom trägt, beschrieben, die über ZNS-Wirkungen verfügen. Weitere 4-Sulfonylaminochroman-Derivate werden in Bioorg. Med. Chem. Lett. 4 (1994), 769 - 773: "N-sulfonamides of benzopyranrelated potassium channel openers: conversion of glyburyde insensitive smooth muscle relaxants to potent smooth muscle contractors" sowie in Trends in Pharm. Sci. 18 (1997), 26 : "Role of the I_{SK} protein in the IminK channel complex", in FEBS Letters 396 (1996), 271-275: "Specific blockade of slowly activating I_{sK} channels by chromanols ..." und Pflügers Arch. - Eur. J. Physiol. 429 (1995), 517-530: "A new class of inhibitors of cAMP-mediated Cl- secretion in rabbit colon, acting by the reduction of cAMP-activated K⁺ conductance" beschrieben.

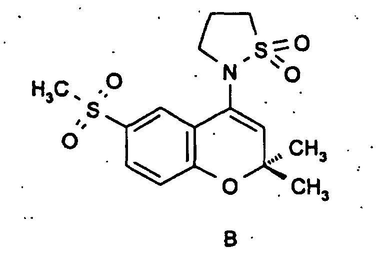

Weitere K⁺-Kanalblocker werden in Biochim. Biophys. Res. 33 (1994), 235: "The novel class III antiarrhythmics NE-10064 and NE-10133 inhibit I_{SK} channels expresssed in xenopus oocytes and I_{KS} in guinea pig cardiac myocytes" sowie in Drug Development Research, 33, Nr. 3 (1994), 235 "Potassium Channel Blockers as Antiarrhythmic Drugs" beschrieben. In den beiden Anmeldungen EP0807629 und EP0860440 werden bestimmte Sufonamid-substituierte Chromane mit K⁺-Kannal blockierender Wirkung beschrieben

Die vorliegende Erfindung betrifft Verbindungen der Formel I, 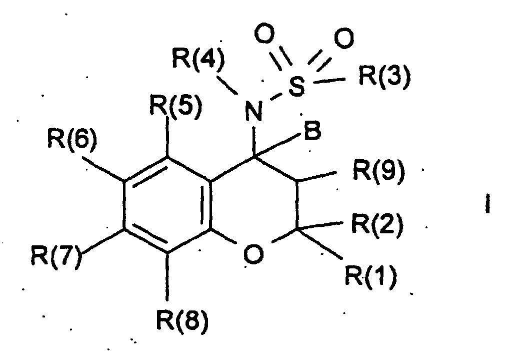 worin bedeuten:
- R(1) und R(2): unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
- R(1) und R(2): gemeinsam eine Alkylenkette mit 2, 3, 4, 5, 6, 7, 8, 9 oder 10 C-Atomen;
- R(3): R(10) -CₙH₂ₙ-NR(11)- oder R(10)-CₙH₂ₙ-,
wobei eine CH₂-Gruppe in den Gruppen CₙH₂ₙ ersetzt sein kann durch -O-, -CO-, -S-, -SO-, -SO₂- oder -NR(12a)-;
R(12a) Wasserstoff, Methyl oder Ethyl;
R(10) Wasserstoff, Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10) und R(11) gemeinsam eine Bindung, sofern n nicht kleiner als 3 ist; oder
- R(3): gemeinsam mit R(4)
eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(12a)- ersetzt sein kann;
R(12a) Wasserstoff, Methyl oder Ethyl;
- R(4): R(13) -CᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, -NR(14)- oder -CONR(14)-;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) H, CF₃, C₂F₅, C₃F₇, Cycloalkyl mit 3, 4, 5, 6, 7 oder 8 C-Atomen, -NR(15)R(16), -CONR(15)R(16), -OR(17), -COOR(17), Phenyl oder Pyrrolyl, Imidazolyl, Chinolyl, Pyrazolyl, Pyridyl, Pyrazinyl, Pyrimidinyl oder Pyridazinyl,
wobei Phenyl und der Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16) unabhängig voneinander Wasserstoff oder Alkyl mit 1, 2, 3 oder 4 C-Atomen;
oder
R(15) und R(16) gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen, - CₓH₂ₓOR(12c);
R(12c) Wasserstoff, Methyl oder Ethyl;
x 2 oder 3;
r Null, 1, 2, 3, 4, 5, 6, 7, 8, 9, 10, 11, 12, 13, 14, 15, 16, 17, 18, 19 oder 20;
- R(6): -Y-CₛH₂ₛ-R(18), Thienyl, Furyl oder Pyrrolyl, Imidazolyl, Chinolyl, Pyrazolyl Pyridyl, Pyrazinyl, Pyrimidinyl oder Pyridazinyl,
wobei der Heterocyclus unsubstituiert sind oder substituiert mit 1 oder 2 Substituenten, ausgewählt aus der Gruppe bestehend aus F Cl, Br, I, CF₃, NO₂, CN, NH₂, OH, Methyl, Ethyl, Methoxy, Methylamino, Dimethylamino, Ethylamino, Diethylamino, Sulfamoyl, Methylsulfonyl und Methylsulfonylamino;
Y -O-, -CO-, -O-CO-, -S-, -SO-, -SO₂-, -SO₂-O-, -SO₂NR(12d)-, -NR(12d)- oder -CONR(12d)-,
wobei die Anknüpfung an den Benzolkem jeweils über das links stehende Atom erfolgt;
R(12d) Wasserstoff, Methyl oder Ethyl;
s 1, 2, 3, 4, 5 oder 6;
R(18) substituiertes Phenyl, das einen oder zwei Substituenten trägt ausgewählt aus der Gruppe bestehend aus NO₂, CN, NH₂, N(Methyl)_{2,} OH, Ethyl, -COOH, -COOMethyl, -COOEthyl, -CONH₂, -CON(Methyl)₂ ;
oder
R(18) Pyrrolyl, Imidazolyl, Chinolyl, Pyrazolyl, Pyridyl, Pyrazinyl, Pyrimidiny oder Pyridazinyl, das einen oder 2 Substituenten trägt ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(18) -OR(19), -SO₂R(19), -NR(19)R(20), -CONR(19)R(20); R(19) und R(20)
unabhängig voneinander CₜH₂ₜ-R(21);
t Null, 1, 2, 3, 4, 5 oder 6;
R(21) Wasserstoff, CF₃, C₂F₅, C₃F₇, Cycloalkyl mit 3, 4, 5, 6, 7 oder 8 C-Atomen, NR(22)R(23), -OR(24), Phenyl, Thienyl, Pyrrolyl, Imidazolyl, Chinolyl, Pyrazolyl, Pyridyl, Pyrazinyl, Pyrimidinyl oder Pyridazinyl
wobei Phenyl, Thienyl und der Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(22) und R(23) unabhängig voneinander Wasserstoff, Alkyl mit 1, 2 oder 3 C-Atomen;
oder
R(22) und R(23) gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(24) Wasserstoff, Alkyl mit 1, 2 oder 3 C-Atomen;
- R(5), R(7) und R(8): unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂, OR(12e) oder NR(12e)R(12f);
R(12e) und R(12f) unabhängig voneinander Wasserstoff oder Alkyl mit 1, 2, 3 oder 4 C-Atomen;
- R(9): Wasserstoff, OR(12g) oder OCOR(12g);
R(12g) Wasserstoff oder Alkyl mit 1, 2 oder 3 C-Atomen;
- B: Wasserstoff;
oder
- R(9) und B: gemeinsam eine Bindung;
sowie ihre physiologisch verträglichen Salze.

Bevorzugt sind Verbindungen der Formel I, worin bedeuten:
- R(1) und R(2): unabhängig voneinander Wasserstoff, CF₃ oder Alkyl mit 1, 2, 3, 4, 5 oder 6 C-Atomen;
oder
- R(1) und R(2): gemeinsam eine Alkylenkette mit 2, 3, 4, 5 oder 6 C-Atomen;
- R(3): R(10)-CₙH₂ₙ-;
R(10) Methyl, CF₃ oder C₂F₅;
n Null, 1 oder 2;
- R(4): R(13)-CᵣH₂ᵣ,
wobei eine CH₂-Gruppe der Gruppe CᵣH₂ᵣ ersetzt sein kann durch =O-, -CH=CH-, -C≡C-, -CO-, -CO-O-, -O-CO-, -S-, -SO-, -SO₂-, -NR(14)- oder -CONR(14)-;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) H, CF₃, C₂F₅, Cycloalkyl mit 3, 4, 5, 6 oder 7 C-Atomen, - NR(15)R(16), -CONR(15)R(16), -OR(17), -COOR(17), Phenyl oder Pyrrolyl, Imidazolyl, Chinolyl, Pyrazolyl, Pyridyl, Pyrazinyl, Pyrimidinyl oder Pyridazinyl,
wobei Phenyl und der Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
R(15) und R(16) unabhängig voneinander Wasserstoff oder Alkyl mit 1, 2, 3 oder 4 C-Atomen;
oder
R(15) und R(16) gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen, - CₓH₂ₓOR(12c);
R(12c) Wasserstoff, Methyl oder Ethyl;
x 2 oder 3;
r 1, 2, 3, 4, 5, 6, 7, 8, 9, 10, 11 oder 12;
- R(6): -Y-C_{S}H₂ₛ-R(18), Thienyl, Furyl oder Pyrrolyl, Imidazolyl, Chinolyl, Pyrazolyl, Pyridyl, Pyrazinyl, Pyrimidinyl oder Pyridazinyl
wobei Thienyl, Furyl und der Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Methylamino, Dimethylamino, Ethylamino, Diethylamino, Sulfamoyl, Methylsulfonyl und Methylsulfonylamino;
Y -O-, -CO-, -O-CO-, -S-, -SO-, -SO₂-, -SO₂-O-, -SO₂NR(12d)-, -NR(12d)- oder -CONR(12d)-,
wobei die Anknüpfung an den Benzolkern jeweils über das links stehende Atom erfolgt;
R(12d) Wasserstoff, Methyl oder Ethyl;
s 1, 2, 3, 4, 5 oder 6;
R(18) substituiertes Phenyl, das einen oder zwei Substituenten trägt ausgewählt aus der Gruppe bestehend aus NH₂, N(Methyl)₂, OH, Ethyl, -COOMethyl, -COOEthyl, -CONH₂, -CON(Methyl)₂ ;
oder
R(18) Pyrrolyl, Imidazolyl, Chinolyl, Pyrazolyl, Pyridyl, Pyrazinyl, Pyrimidinyl oder Pyridazinyl, die einen oder 2 Substituenten tragen ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
oder
R(18) -OR(19), -NR(19)R(20), -CONR(19)R(20);
R(19) und R(20) unabhängig voneinander CₜH₂ₜ-R(21);
t Null, 1, 2, 3, 4, 5 oder 6;
R(21) Wasserstoff, CF₃, NR(22)R(23), -OR(24), Phenyl, Thienyl, Pyrrolyl, Imidazolyl, Chinolyl, Pyrazolyl, Pyridyl, Pyrazinyl, Pyrimidinyl oder Pyridazinyl,
wobei Phenyl, Thienyl und der Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
R(22) und R(23) unabhängig voneinander Wasserstoff, Alkyl mit 1, 2 oder 3 C-Atomen;
oder
R(22) und R(23) gemeinsam eine Kette von 4 oder 5 Methylengruppen, von denen eine CH₂-Gruppe durch -O-, -S-, -NH- oder -N(CH₃)-ersetzt sein kann;
R(24) Wasserstoff, Alkyl mit 1, 2 oder 3 C-Atomen;
- R(5), R(7) und R(8): unabhängig voneinander Wasserstoff, F, Cl, Br, Alkyl mit 1, 2, 3, 4 oder 5 C-Atomen, CN, CF₃, NO₂, OR(12e);
R(12e) Alkyl mit 1, 2, 3 oder 4 C-Atomen;
- R(9): Wasserstoff oder OH;
- B: Wasserstoff;
oder
- R(9) und B: gemeinsam eine Bindung;
sowie ihre physiologisch verträglichen Salze.

Bevorzugt sind Verbindungen der Formel I, worin bedeuten:
- R(1) und R(2): unabhängig voneinander Wasserstoff, CF₃ oder Alkyl mit 1 oder 2 C-Atomen;
oder
- R(1) und R(2): gemeinsam eine Alkylenkette mit 2, 3, 4 oder 5 C-Atomen;
- R(3): Methyl oder Ethyl;
- R(4): R(13)-CᵣH₂ᵣ,
wobei eine CH₂-Gruppe der Gruppe CᵣH₂ᵣ ersetzt son kann durch -O-, -CO-O-, -O-CO-, -NR(14)- oder -CONR(14)-;
R(14) Wasserstoff oder Alkyl mit 1 oder 2 C-Atomen;
R(13) Wasserstoff, CF₃, -NR(15)R(16), -CONR(15)R(16), -OR(17), - COOR(17), Phenyl, Pyrrolyl, Imidazolyl, Chinolyl, Pyrazolyl, Pyridyl, Pyrazinyl, Pyrimidinyl oder Pyridazinyl,
wobei Phenyl und der Heterocyclus unsubstituiert sind oder substituiert mit 1 oder 2 Substituenten, ausgewählt aus der Gruppe bestehend aus F, Cl, Br, CF₃, Methyl, Methoxy, Sulfamoyl, Methylsulfonyl und Methylsulfonylamino;
R(15) und R(16) unabhängig voneinander Wasserstoff oder Alkyl mit 1 oder 2 C-Atomen;
oder
R(15) und R(16) gemeinsam eine Kette von 4 oder 5 Methylengruppen, von denen eine CH₂-Gruppe durch -O-, -NH- oder -N(CH₃)-ersetzt sein kann;
R(17) Wasserstoff oder Alkyl mit 1 oder 2 C-Atomen;
r 1, 2, 3, 4, 5, 6 oder 7;
- R(6): -Y-CₛH₂ₛ-R(18), Thienyl, Furyl, Pyrrolyl, Imidazolyl, Chinolyl, Pyrazolyl, Pyridyl, Pyrazinyl, Pyrimidinyl oder Pyridazinyl
wobei Thienyl, Furyl und der Heterocyclus unsubstituiert sind oder substituiert mit 1 oder 2 Substituenten, ausgewählt aus der Gruppe bestehend aus F, Cl, Br, CF₃, Methyl, Methoxy, Methylamino, Dimethylamino, Ethylamino, Diethylamino, Sulfamoyl, Methylsulfonyl und Methylsulfonylamino;
Y -O- oder -CONR(12d)-,
wobei die Anknüpfung an den Benzolkern über das links stehende Atom erfolgt;
R(12d) Wasserstoff, Methyl oder Ethyl;
s 1, 2, 3, 4, 5 oder 6;
R(18) substituiertes Phenyl, das einen oder zwei Substituenten trägt ausgewählt aus der Gruppe bestehend aus NH₂, N(Methyl)₂, OH, -COOMethyl, -COOEthyl, -CON(Methyl)₂;
oder
R(18) Pyrrolyl, Imidazolyl, Chinolyl, Pyrazolyl, Pyridyl, Pyrazinyl, Pyrimidinyl oder Pyridazinyl, das einen oder 2 Substituenten trägt ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
oder
R(18) -OR(19) oder -CONR(19)R(20);
R(19) und R(20) unabhängig voneinander CₜH₂ₜ-R(21);
t Null, 1, 2 oder 3;
R(21) Wasserstoff, CF₃, NR(22)R(23), -OR(24);
R(22) und R(23) unabhängig voneinander Wasserstoff, Alkyl mit 1, 2 oder 3 C-Atomen;
oder
R(22) und R(23) gemeinsam eine Kette von 4 oder 5 Methylengruppen, von denen eine CH₂-Gruppe durch -O-, -S-, -NH- oder -N(CH₃)-ersetzt sein kann;
R(24) Wasserstoff, Alkyl mit 1 oder 2 C-Atomen;
- R(5), R(7) und R(8): Wasserstoff;
- R(9): Wasserstoff oder OH;
- B: Wasserstoff;
oder
- R(9) und B: gemeinsam eine Bindung;
sowie ihre physiologisch verträglichen Salze.

Ganz besonders bevorzugt sind Verbindungen der Formel I, worin bedeuten:
- R(1) und R(2): Methyl;
- R(3): Methyl oder Ethyl;
- R(4): R(13)-CᵣH₂ᵣ,
wobei eine CH₂-Gruppe der Gruppe CᵣH₂ᵣ ersetzt sein kann durch -O-;
R(13) Wasserstoff, CF₃;
r 1, 2, 3, 4, 5 oder 6;
- R(6): -Y-CₛH₂ₛ-R(18), Thienyl, Pyrrolyl, Imidazolyl, Chinolyl, Pyrazolyl, Pyridyl, Pyrazinyl, Pyrimidinyl oder Pyridazinyl,
wobei Thienyl und der Heterocyclus unsubstituiert sind oder substituiert mit 1 oder 2 Substituenten, ausgewählt aus der Gruppe bestehend aus F, Cl, CF₃, Methyl, Methoxy, Dimethylamino, Sulfamoyl, Methylsulfonyl und Methylsulfonylamino;
Y -O- ;
s 1, 2, 3, 4, 5 oder 6;
R(18) Pyrrolyl, Imidazolyl, Chinolyl, Pyrazolyl, Pyridyl, Pyrazinyl, Pyrimidinyl oder Pyridazinyl, das einen oder 2 Substituenten trägt ausgewählt aus der Gruppe bestehend aus F, Cl, CF₃, NO₂, CN, OH, Methyl, Methoxy, Dimethylamino, Sulfamoyl, Methylsulfonyl und Methylsulfonylamino;
oder
R(18) -OR(19) oder -CONR(19)R(20);
R(19) und R(20) unabhängig voneinander CₜH₂ₜ-R(21);
t Null, 1, 2 oder 3;
R(21) Wasserstoff, CF₃, NR(22)R(23), -OR(24);
R(22) und R(23) unabhängig voneinander Wasserstoff oder Alkyl mit 1 oder 2 C-Atomen;
R(24) Wasserstoff oder Alkyl mit 1 oder 2 C-Atomen;
- R(5), R(7) und R(8): Wasserstoff;
- R(9): Wasserstoff;
- B: Wasserstoff;

Alkylreste und Alkylenreste können geradkettig oder verzweigt sein. Dies gilt auch für die Alkylenreste der Formeln CᵣH₂ᵣ, CₜH₂ₜ, CₙH₂ₙ und CₛH₂ₛ. Alkylreste und Alkylenreste können auch geradkettig oder verzweigt sein, wenn sie substituiert sind oder in anderen Resten enthalten sind, z. B. in einem Alkoxyrest oder in einem Alkylmercaptorest oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,4-Butylen, 1,5-Pentylen, 2,2-Dimethyl-1,3-propylen, 1,6-Hexylen, usw. sind Beispiele für Alkylenreste.

Pyrrolyl ist zum Beispiel 1-, 2- oder 3- Pyrrolyl. Imidazolyl ist zum Beispiel 1-, 2-, 4-oder 5-Imidazolyl. Pyrazolyl ist zum Beispiel 1-, 3-, 4- oder 5-Pyrazolyl. Pyridyl ist zum Beispiel 2-, 3- oder 4-Pyridyl. Pyrimidinyl ist zum Beispiel 2-, 4-, 5- oder 6-Pyrimidinyl. Pyridazinyl ist zum Beispiel 3- oder 4-Pyridazinyl. Chinolyl ist zum Beispiel 2-, 3-, 4-, 5-, 6-, 7- oder 8-Chinolyl.

Thienyl steht sowohl für 2- als auch 3-Thienyl. Furyl steht für 2- und 3-Furyl.

Monosubstituierte Phenylreste können in der 2-, der 3- oderder 4-Position substituiert sein, disubstituierte in der 2,3-, 2,4-, 2,5-, 2,6-, 3,4- oder 3,5-Position. Entsprechendes gilt sinngemäß analog auch für die N-haltigen Heterocylen oder den Thiophenrest.

Bei Disubstitution eines Restes können die Substituenten gleich oder verschieden sein.

Bedeuten die Reste R(1) und R(2) gemeinsam eine Alkylenkette, so bilden diese Reste mit dem sie tragenden Kohlenstoffatom einen Ring, der mit dem 6-Ring in der Formel I ein Kohlenstoffatom gemeinsam hat, es liegt dann also eine Spiroverbindung vor. Bedeuten R(9) und B gemeinsam eine Bindung so liegt ein 2H-Chromengrundgerüst vor. Bedeuten R(10) und R(11) gemeinsam eine Bindung, so steht die Gruppe R(10)-CₙH₂ₙ-NR(11)- bevorzugt für einen über ein Stickstoffatom gebundenen Stickstoffheterocyclus. Wenn R(10) und R(11) gemeinsam eine Bindung bedeuten und die Gruppe R(10)-CₙH₂ₙ-NR(11)- für einen über ein Stickstoffatom gebundenen Stickstoffheterocyclus steht, ist dieser Stickstoffheterocyclus bevorzugt ein 4-Ring oder ein größerer Ring als ein 4-Ring, z. B. ein 5-Ring, 6-Ring oder 7-Ring.

Enthalten die Verbindungen der Formel I eine oder mehrere saure oder basische Gruppen bzw. einen oder mehrere basische Heterocyclen, so sind auch die entsprechenden physiologisch oder toxikologisch verträglichen Salze Gegenstand der Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. sind also in enantiomerenreiner Form, sowohl als links- als auch als rechtsdrehende Antipoden, und auch in Form von Mischungen der beiden Enantiomeren in unterschiedlichen Verhältnissen oder in Form von Racematen Gegenstand der Erfindung. Bei Vorliegen einer cis/trans-Isomerie sind sowohl die cis-Form als auch die trans-Form und Gemische dieser Formen Gegenstand der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Die Verbindungen der Formel I sind durch unterschiedliche chemische Verfahren herstellbar, die ebenfalls Gegenstand der vorliegenden Erfindung sind. So erhält man beispielsweise eine Verbindung der Formel I, indem man
a) eine Verbindung der Formel II, 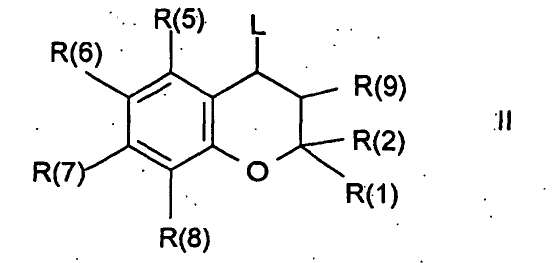 worin R(1), R(2), R(5), R(6), R(7), R(8) und R(9) die oben angegebenen Bedeutungen besitzen und L eine nucleofuge Fluchtgruppe, insbesondere Cl, Br, I, Methansulfonyloxy, Trifluormethansulfonyloxy oder p-Toluolsulfonyloxy, bedeutet, in an sich bekannter Weise umsetzt mit einem Sulfonamid oder dessen Salz der Formel III, 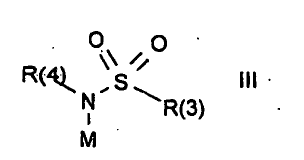 worin R(3) und R(4) die oben angegebenen Bedeutungen besitzen und M für Wasserstoff oder vorzugsweise für ein Metalläquivalent, besonders bevorzugt für Lithium, Natrium oder Kalium steht;
   oder daß man
b) eine Verbindung der Formel IV 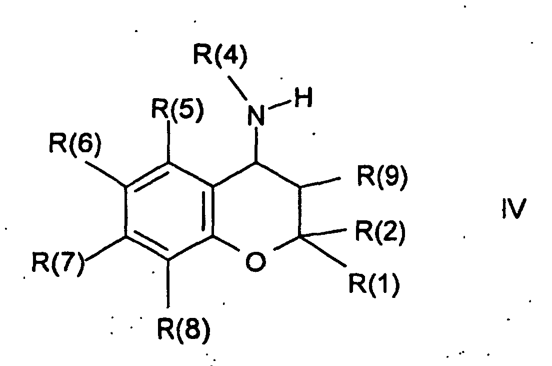 worin R(1), R(2), R(4), R(5), R(6), R(7), R(8) und R(9) die oben angegebenen Bedeutungen besitzen, mit einem Suffonsäure-Derivat der Formel V 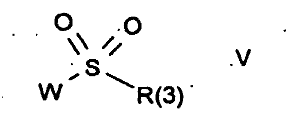 umsetzt, worin R(3) die oben angegebenen Bedeutungen besitzt und W eine nucleofuge. Fluchtgruppe, wie z. B. Fluor, Brom, 1-Imidazolyl, insbesondere aber Chlor, bedeutet;
   oder daß man
c) eine Verbindung der Formel VI 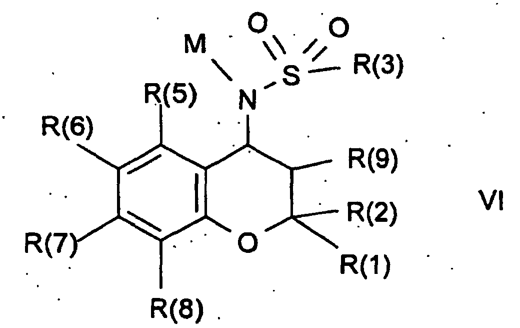 worin R(1), R(2), R(3), R(5), R(6), R(7), R(8), R(9) und M die oben angegebenen Bedeutungen besitzen, in an sich bekannter Weise im Sinne einer Alkylierungsreaktion mit einem Alkylierungsmittel der Formel VII umsetzt,

   R(4)-L VII

   worin R(4) und L die oben angegebenen Bedeutungen besitzen;
   oder daß man
d) in einer Verbindung der Formel I 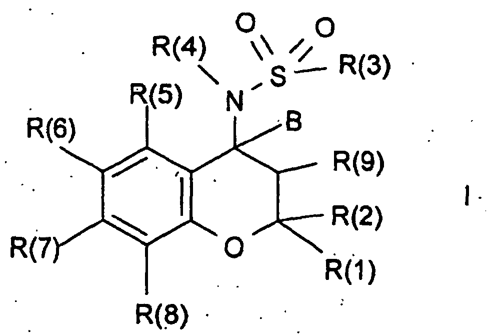 worin R(1) bis R(9) und B die oben angegebenen Bedeutungen besitzen, in mindestens einer der Positionen R(5), R(6), R(7) und R(8) eine elektrophile Substitutionsreaktion durchführt, sofern diese Position Wasserstoff bedeutet;
   oder daß man
e) eine Verbindung der Formel VIII 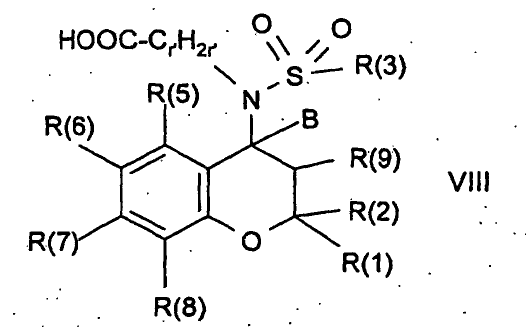 worin R(1), R(2), R(3), R(5), R(6), R(7), R(8), R(9) und B die oben angegebenen Bedeutungen besitzen und r' 1 bis 9 bedeutet, mit einer Verbindung der Formel IX
   oder X 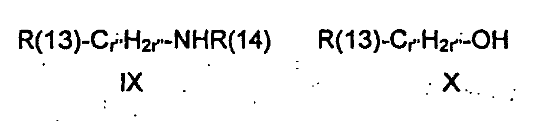 worin R(13) und R(14) die oben angegebenen Bedeutungen besitzen und r" 1 bis 9 bedeutet, umsetzt im Sinne einer Veresterungs- bzw. Amidierungsreaktion;
   oder daß man
f) eine Verbindung der Formel XII 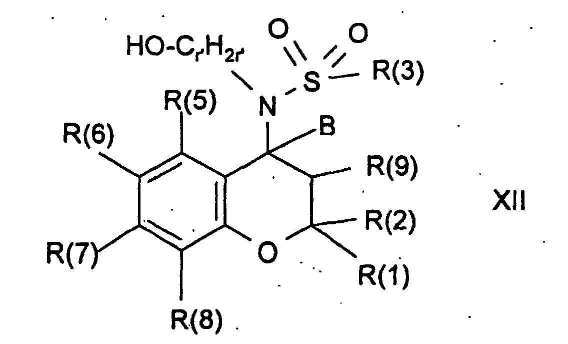 worin R(1), R(2), R(3), R(5), R(6), R(7), R(8), R(9), r' und B die oben angegebenen Bedeutungen besitzen, mit einer Verbindung der Formel XIII

   R(13)-C_{r"}H_{2r"}-L XIII

   worin R(13), r" und L die oben angegebenen Bedeutungen besitzen, umsetzt im Sinne einer Alkylierungsreaktion;
   oder daß man
g) eine Verbindung der Formel XIV 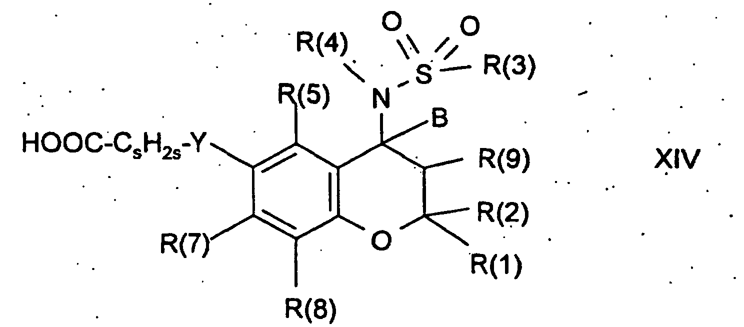 worin R(1), R(2), R(3), R(4), R(5), R(7), R(8), R(9), Y, s und B die oben angegebenen Bedeutungen besitzen, mit einer Verbindung der Formel HNR(19)R(20),
   worin R(19) und R(20) die oben angegebenen Bedeutungen besitzen, umsetzt im Sinne einer Amidierungsreaktion;
   oder daß man
h) eine Verbindung der Formel XV 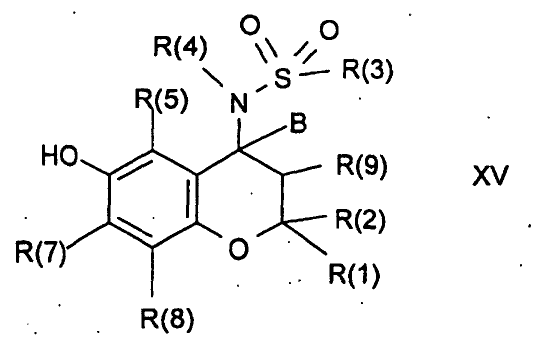 worin R(1), R(2), R(3), R(4), R(5), R(7), R(8), R(9) und B die oben angegebenen Bedeutungen besitzen, mit einer Verbindung der Formel R(18)-CₛH₂ₛ-L, worin R(18), s und L die oben angegebenen Bedeutungen besitzen, umsetzt im Sinne einer Alkylierungsreaktion;
   oder daß man
i) eine Verbindung der Formel XVI 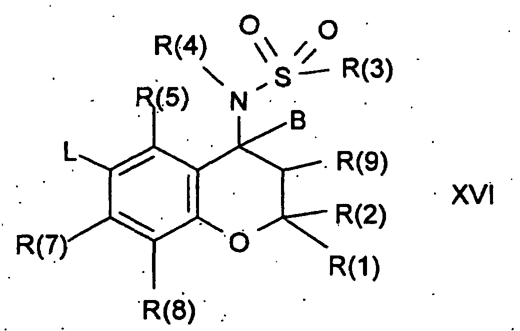 worin R(1), R(2), R(3), R(4), R(5), R(7), R(8), R(9), L und B die oben angegebenen Bedeutungen besitzen, mit einer Verbindung der Formel Het-Met, worin Het für einen N-haltigen Heterocyclus mit 1, 2, 3, 4, 5, 6, 7, 8 oder 9 C-Atomen sowie Thienyl oder Furyl steht und Met B(OH)₂, Trialkylsilyl, ein Alkalimetallkation oder einen leicht substituierbaren organometallischen Rest bedeutet, umsetzt im Sinne einer Kupplungsreaktion;
   oder daß man
j) eine Verbindung der Formel XVII, 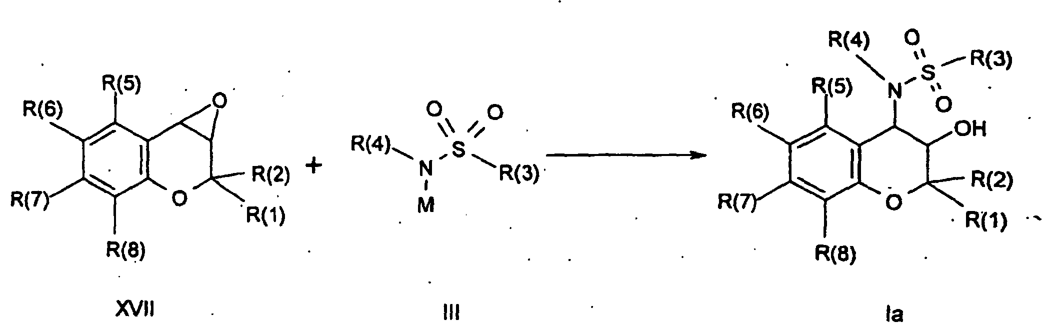 worin R(1), R(2), R(5), R(6), R(7) und R(8) die oben angegebenen Bedeutungen besitzen, mit einem Sulfonamid der Formel III, worin R(3), R(4) und M die oben angegebenen Bedeutungen haben oder M vorteilhäft auch für einen Trialkylsilylrest, z.B. einen Trimethylsilylrest, steht, umsetzt zu einem Chromanol der Formel Ia;
   oder daß man
k) eine Verbindung der Formel Ia, 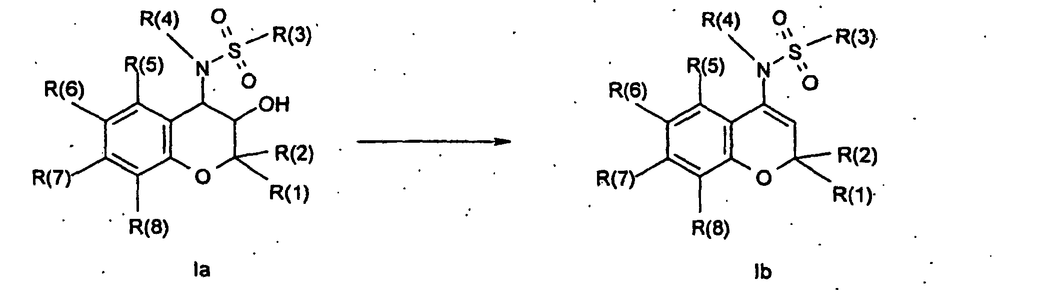
worin R(1) bis R(8) die oben angegebenen Bedeutungen haben, im Sinne einer Eliminierungsreaktion zu einer Verbindung der Formel I b, worin R(1) bis R(8) die oben angegebenen Bedeutungen haben, umwandelt.

Die Verfahrensweise a) entspricht der nucleophilen Substitution einer Abgangsgruppe in einem reaktiven Bicyclus der Formel II durch ein Sulfonamid bzw. eines seiner Salze der Formel III. Wegen der höheren Nucleophilie und höheren Reaktivität eines in der Salzform vorliegenden Sulfonamids ist bei Verwendung eines freien Sulfonamids (Formel III, M = H) bevorzugt, aus diesem zunächst durch Einwirkung einer Base ein Sulfonamidsalz (Formel III, M = Metallkation) zu erzeugen. Setzt man ein freies Sulfonamid (Formel III, M = H) ein, so kann die Deprotonierung des Sulfonamids zum Salz in situ erfolgen. Bevorzugt werden solche Basen verwandt, die selbst nicht oder nur wenig alkyliert werden, wie z. B. Natriumcarbonat, Kaliumcarbonat, sterisch stark gehinderte Amine, z. B. Dicyclohexylamin, N,N-Dicyclohexyl-ethylamin, oder andere starke Stickstoffbasen mit geringer Nucleophilie, beispielsweise DBU (Diazabicycloundecen), N,N',N'''-Triisopropylguanidin etc. Es können allerdings auch andere üblich verwendete Basen für die Reaktion eingesetzt werden, wie Kalium-tert-butylat, Natriummethylat, Alkalihydrogencarbonate, Alkalihydroxide, wie beispielsweise LiOH, NaOH oder KOH, oder Erdalkalihydroxide, wie beispielsweise Ca(OH)₂ .

Vorzugsweise arbeitet man in einem Lösungsmittel, besonders bevorzugt in polaren organischen Lösungsmitteln wie z. B. Dimethylformamid (DMF), Dimethylacetamid (DMA), Dimethylsulfoxid (DMSO), Tetramethylharnstoff (TMU), Hexamethylphosphorsäuretriamid (HMPT), Tetrahydrofuran (THF), Dimethoxyethan (DME) oder anderen Ethem, oder beispielsweise auch in einem Kohlenwasserstoffwie Toluol oder in einem halogenierten Kohlenwasserstoff wie Chloroform oder Methylenchlorid usw. Es kann aber auch in polaren protischen Lösungsmitteln gearbeitet werden, wie z. B. in Wasser, Methanol, Ethanol, Isopropanol, Ethylenglykol oder dessen Oligomeren und deren entsprechenden Hälbethern oder auch deren Ethern. Die Reaktion kann auch in Gemischen dieser Lösungsmittel durchgeführt werden. Ebenso kann die Reaktion aber auch ganz ohne Lösungsmittel durchgeführt werden. Die. Reaktion wird bevorzugt in einem Temperaturbereich von -10 bis +140 °C, besonders bevorzugt im Bereich von 20 bis 100 °C durchgeführt. In günstiger Weise kann Verfahrensweise a) auch unter den Bedingungen einer Phasentransferkatalyse durchgeführt werden.

Die Verbindungen der Formel II gewinnt man nach literaturbekannten Methoden, beispielsweise aus den entsprechenden Alkoholen (Formel II, L = -OH) durch Einwirkung von Hatogenwasserstoff HL (L = Cl, Br, I) oder durch Einwirkung eines anorganischen Säurehalogenids (POCl₃, PCl₃, PCl₅, SOCl₂, SOBr₂) oder durch radikalische Halogenierung der entsprechenden Chroman-Derivate (Formel II, L = H) mit elementarem Chlor oder Brom, oder mit radikalisch aktivierbaren Halogenierungsmitteln wie N-Bromsuccinimid (NBS) oder SO₂Cl₂ (Sulfurylchlorid) in Gegenwart eines Radikalkettenstarters wie energiereichem Licht des sichtbaren oder ultravioletten Wellenbereiches oder durch Verwendung eines chemischen Radikalstarters wie Azodiisobutyronitril.

### Verfahrensweise b)

beschreibt die an sich bekannte und häufig angewandte Reaktion einer reaktiven Sulfonylverbindung der Formel V, insbesondere einer Chlorsulfonylverbindung (W = Cl), mit einem Amino-Derivat der Formel IV zum entsprechenden Sulfonamid-Derivat der Formel I. Die Reaktion kann prinzipiell ohne Lösungsmittel durchgeführt werden, jedoch werden derartige Reaktionen in den meisten Fällen unter Verwendung eines Lösungsmittels durchgeführt.

Die Reaktionsführung geschieht vorzugsweise unter Verwendung eines polaren Lösungsmittels vorzugsweise in Gegenwart einer Base, die selbst vorteilhaft als Lösungsmittel verwendet werden kann, z.B. bei Verwendung von Triethylamin, insbesondere von Pyridin und dessen Homologen. Ebenfalls verwendete Lösungsmittel sind beispielsweise Wasser, aliphatische Alkohole, z.B. Methanol, Ethanol, Isopropanol, sek. Butanol, Ethylenglykol und dessen monomere und oligomere Monoalkyl- und Dialkylether, Tetrahydrofuran, Dioxan, dialkylierte Amide wie DMF, DMA, sowie TMU und HMPT. Man arbeitet dabei bei einer Temperatur von 0 bis 160°C, vorzugsweise von 20 bis 100°C.

Die Amine der Formel IV erhält man in literaturbekannter Weise bevorzugt aus den entsprechenden Carbonylverbindungen der Formel XX, 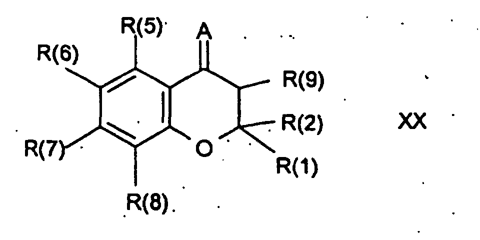 worin R(1), R(2), R(5), R(6), R(7), R(8) und R(9) die oben angegebenen Bedeutungen besitzen und A Sauerstoff bedeutet, entweder mit Ammoniak oder einem Amin der Formel XXI,

R(4)-NH₂ XXI

in der R(4) die angegebenen Bedeutungen hat, unter reduktiven Bedingungen oder reduktiven katalytischen Bedingungen, vorzugsweise bei höherer Temperatur und im Autoklaven. Dabei werden primär durch Kondensationsreaktion der Ketone der Formel XX (A = Sauerstoff) und der Amine der Formel XXI in situ Schiff-Basen der Formel XX, in der A für R(4)-N= steht, gebildet, die unmittelbar, d. h. ohne vorherige Isolierung, reduktiv in die Amine der Formel IV überführt werden können. Es können aber auch die bei der Kondensationsreaktion intermediär aus den Verbindungen der Formel XX und XXI entstehenden Schiff-Basen (Formel XX, A steht für R(4)-N=) nach literaturbekannten Methoden hergestellt und zunächst isoliert werden, um sie dann in einem separaten Schritt mit einem geeigneten Reduktionsmittel, wie z. B. NaBH₄, LiAlH₄, NaBH₃CN oder durch katalytische Hydrierung in Gegenwart von z. B. Raney-Nickel oder einem Edelmetall wie z. B. Palladium, in die Verbindungen der Formel IV umzuwandeln.

Die Verbindungen der Formel IV, in der R(4) für Wasserstoff steht, können vorteilhaft in literaturbekannter Weise auch durch Reduktion von Oximen oder Oximethem (Formel XX, A steht für =N-OR, R = H oder Alkyl) oder Hydrazonen (Formel XX, A steht für =N-NR₂, R z. B. = H oder Alkyl) erhalten werden, z. B. unter Verwendung eines komplexen Metallhydrids oder durch katalytische Hydrierung. Die dafür erforderlichen Oxime und Hydrazone werden vorzugsweise in an sich bekannter Weise aus den Ketonen der Formel XX (A = Sauerstoff) mit Hydrazin oder einem seiner Derivate oder beispielsweise mit Hydroxylamin-Hydrochlorid unter wasserabspaltenden Bedingungen hergestellt. Besonders vorteilhaft können die Verbindungen der Formel IV, in der R(4) für Wasserstoff steht, auch durch Aminierung mit einer geeigneten Ammoniumverbindung, z.B. Ammoniumacetat, in Gegenwart eines geeigneten Reduktionsmittels, wie z.B. NaCNBH₃, erhalten werden (J. Am. Chem. Soc. 93, 1971, 2897).

Alternativ können die Amino-Derivate der Formel IV auch in an sich. literaturbekannter Weise durch Reaktion der reaktiven Verbindungen der Formel II mit R(1), R(2), R(5), R(6), R(7), R(8), R(9) und L in der angegebenen Bedeutung, entweder mit Ammoniak oder einem Amin der Formel XXI, mit R(4) in der angegebenen Bedeutung, erhalten werden.

### Verfahrensweise c)

repräsentiert die an sich bekannte Alkylierungsreaktion eines Sulfonamids bzw. eines seiner Salze VI mit einem Alkylierungsmittel der Formel VII. Entsprechend der Reaktionsanalogie mit Verfahrensweise a) gelten für Verfahrensweise c) die bereits ausführlich unter Verfahrensweise a) beschriebenen Reaktionsbedingungen. Neben den dort bereits genannten Basen werden vorzugsweise Natriumhydrid oder eine Phosphazenbase zur Deprotonierung des Sulfonamids. eingesetzt.

Die Herstellung der Sulfonamid-Derivate VI (mit M=H) und deren Vorprodukte wurden bereits bei Verfahrensweise b) beschrieben, wobei R(4) dann jeweils Wasserstoff bedeutet. Die Herstellung der Alkylantien VII erfolgt nach Analogvorschriften der Literatur bzw. wie unter Verfahrensweise a) beschrieben, vorzugsweise aus den entsprechenden Hydroxyverbindungen (Formel VII mit L gleich -OH).

### Verfahrensweise d)

beschreibt die weitere chemische Umwandlung von erfindungsgemäßen Verbindungen der Formel I in andere Verbindungen der Formel I durch elektrophile Substitutionsreaktionen in einer oder in mehreren der mit R(5) bis R(8) bezeichneten Positionen, die jeweils Wasserstoff bedeuten.

Bevorzugte Substitütionsreaktionen sind
1. die aromatische Nitrierung zur Einführung einer oder mehrerer Nitrogruppen, die in nachfolgenden Reaktionen teilweise oder alle zu Aminogruppen reduziert werden können. Die Aminogruppen können wiederum in nachfolgenden Reaktionen in andere Gruppen umgewandelt werden, beispielsweise in einer Sandmeyerreaktion, z. B. zur Einführung von Cyanogruppen;
2. die aromatische Halogenierung insbesondere zur Einführung von Chlor, Brom oder Jod;
3. die Chlorsulfonierung, z.B. durch Einwirkung von Chlorsulfonsäure, zur Einführung einer Chlorsulfonylgruppe, die in nachfolgenden Reaktionen in andere Gruppen umgewandelt werden kann, z. B. in eine Sulfonamidgruppe;
4. die Friedel-Crafts-Acylierungsreaktion zur Einführung eines Acylrestes oder eines Sulfonylrestes durch Einwirkung der entsprechenden Säurechloride in Gegenwart einer Lewis-Säure als Friedel-Crafts-Katalysator, vorzugsweise in Gegenwart von wasserfreiem Aluminiumchlorid.

### Verfahrensweise e)

beschreibt die Veresterung von Carbonsäuren der Formel VIII mit Alkoholen der Formel X bzw. die Amidierung mit Aminen der Formel IX. Für diese Reaktionen sind in der Literatur zahlreiche Methoden beschrieben worden. Besonders vorteilhaft können diese Reaktionen durch Aktivierung der Carbonsäure, z.B. mit Dicyclohexylcarbodiimid (DCC), gegebenenfalls unter Zusatz von Hydroxybenzotriazol (HOBT) oder Dimethylaminopyridin (DMAP), oder mit O-[(Cyano(ethoxycarbonyl)methylen)amino]-1,1,3,3-tetramethyluroniumtetrafluoroborat (TOTU), durchgeführt werden. Es können aber auch zunächst nach bekannten Methoden reaktive Säurederivate synthetisiert werden, z.B. Säurechloride durch Umsetzung der Carbonsäuren der Formel VIII mit anorganischen Säurehalogeniden, wie z.B. SOCl₂, oder Säureimidazolide durch Umsetzung mit Carbonyldiimidazol, die dann anschließend, gegebenenfalls unter Zusatz einer Hilfsbase, mit den Alkoholen bzw. Aminen der Formeln X oder IX umgesetzt werden.
Die Carbonsäuren der Formel VIII erhält man nach den unter a) bis d) beschriebenen Methoden, wobei dann jedoch R(4) jeweils -C_{r'}H_{2r'}COOH bzw. - C_{r'}H_{2r'}COOAlkyl bedeutet und im letzteren Fall noch eine anschließende Verseifung des Esters erfolgt.

### Verfahrensweise f)

beschreibt die Alkylierung eines Alkohols der Formel XII mit einem Alkylierungsmittel der Formel XIII. Hierzu wird der Alkohol zunächst durch Einwirkung einer geeigneten Base, wie z.B. Natriumhydrid oder eine Phosphazenbase, in ein Alkoholatsalz überführt, das dann in einem geeigneten polaren Lösungsmittel, wie z.B. Dimethylformamid, bei Temperaturen zwischen 20 und 150°C mit dem Alkylierungsmittel umgesetzt wird. Die Deprotonierung des Alkohols zum Salz kann auch in situ erfolgen, wobei dann bevorzugt Basen eingesetzt werden, die selbst nicht alkyliert werden, wie z.B. Kaliumcarbonat.
Die Alkohole der Formel XII erhält man nach den unter a) bis d) beschriebenen Methoden, wobei dann jedoch R(4) jeweils -C_{r'}H_{2r'}OH bzw. -C_{r'}H_{2r'}OR (R=geeignete Schutzgruppe, z.B. Acetoxy) bedeutet und im letzteren Fall noch eine anschließende Abspaltung der Schutzgruppe erfolgt. Die Alkohole der Formel XII können aber auch durch Reduktion der unter Verfahrensweise e) beschriebenen Ester der Formel I, bei denen R(4) -C_{r'}H_{2r'}COOAlkyl bedeutet, z.B. mit Lithiumaluminiumhydrid, erhalten werden.

### Verfahrensweise g)

beschreibt die Amidierung von Carbonsäuren der Formel XIV mit Aminen der Formel HNR(19)R(20), die unter den bei Verfahrensweise e) angegebenen Reaktionsbedingungen durchgeführt werden kann. Die Carbonsäuren der Formel XIV erhält man z.B. analog der unter Verfahrensweise h) beschriebenen Methode, wobei dann jedoch R(18) für COOH bzw. COOAlkyl steht und im letzteren Fall noch eine anschließende Verseifung des Esters erfolgt.

### Verfahrensweise h)

entspricht der Alkylierung eines Phenols der Formel XV mit einem Alkylierungsmittel der Formel R(18)-CₛH₂ₛ-L, die unter den bereits bei Verfahrensweise f) beschriebenen Reaktionsbedingungen durchgeführt werden kann. Die Phenole der Formel XV können nach den unter a) bis f) beschriebenen Verfahrensweisen erhalten werden, wobei dann jedoch R(6) jeweils für eine OH-Gruppe oder ein entsprechend geschütztes Derivat, z.B. einen Benzylether, steht und anschließend noch eine Abspaltung der Schutzgruppe erfolgt.

### Verfahrensweise i)

.beschreibt die Kopplung eines Arylhalogenids, z.B. -iodids, oder eines Arylalkylsulfonats, z.B. -triflates, der Formel XVI mit einem Heterocyclus der Formel Het-Met in Gegenwart eines geeigneten Übergangsmetallkatalysators. Bevorzugt werden Heterocyclen eingesetzt, bei denen die Gruppe Met für einen Boronsäurerest, z.B. B(OH)₂, steht, die im Sinne einer Suzuki-Kopplung mit Arylhalogeniden der Formel XVI, z.B. in Gegenwart von Palladiumtetrakis(triphenylphosphin) und einer Base wie z.B. Kaliumcarbonat oder Cäsiumcarbonat, umgesetzt werden können. Es können aber auch Heterocyclen eingesetzt werden, bei denen die Gruppe Met z.B. für einen Trialkylzinnrest (Stille-Kopplung) oder einen Trialkylsilylrest steht oder auch Grignard- oder Zinkorganische Verbindungen. Entsprechende Reaktionsbedingungen für derartige Kopplungen sind in der Literatur beschrieben.

### Verfahrensweise j)

entspricht der nucleophilen Öffnung eines Epoxids der Formel XVII durch ein Sulfonamid bzw. eines seiner Salze der Formel III. Die Reaktion kann unter analogen Bedingungen wie für Verfahrensweise a) beschrieben durchgeführt werden. Als besonders vorteilhaft hat sich die Verwendung des freien Sulfonamids in Gegenwart eines Unterschusses, z.B. 20 - 80%, der entsprechenden Base, z.B. Natriumhydrid, erwiesen. Ebenfalls vorteilhaft ist die Verwendung von Sutfonamidderivaten, bei denen M für einen Trialkylsilyl-, z.B. einen Trirnethylsitylrest, steht, wobei es dann zweckmäßig ist, die Reaktion in Gegenwart eines Fluorids, z.B. Tetrabutylammoniumfluorid, durchzuführen.

Die Epoxide der Formel XVII erhält man nach literaturbekannten Methoden aus den entsprechenden Olefinen der Formel XXII, 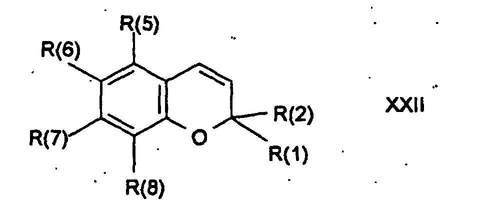 worin R(1), R(2), R(5), R(6), R(7) und R(8) die oben angegebenen Bedeutungen besitzen, z. B. durch Einwirkung eines geeigneten anorganischen oder organischen Peroxids, wie beispielsweise H₂O₂ oder m-Chlorperbenzoesäure, oder durch basenkatalysierte Cyclisierung des entsprechenden Bromhydrins, das aus XXII z. B. durch Umsetzung mit N-Bromsuccinimid und Wasser erhalten werden kann. Die Epoxide der Formel XVII können aus den Olefinen der Formel XXII auch in optisch reiner Form erhalten werden durch Oxidation in Gegenwart des chiralen Jacobsen-Katalysators, wie z.B. in Tetrahedron Lett. 32, 1991, 5055 beschrieben ist. Die Olefine der Formel XXII lassen sich erhalten entweder aus den Ketonen der Formel XX (A=Sauerstoff) durch Reduktion der Carbonylgruppe zu einer OH-Funktion und anschließende säurekatalysierte Eliminierung oder durch thermische Cyclisierung von geeignet substituierten Aryl-propargylethern, wie z.B. in J. Org. Chem. 38 (1973) 3832 beschrieben.

### Verfahrensweise k)

beschreibt die Überführung eines Chromanols der Formel la in ein Chromen der Formel Ib durch Eliminierung. Hierzu kann das Chromanol entweder direkt in Gegenwart einer Säure oder Base einer Wasserabspaltung unterworfen werden, oder es kann zunächst eine Aktivierung der Hydroxygruppe erfolgen, z.B. durch Acetylierung mitAcetanhydrid oder Mesylierung mit Methansulfonsäurechlorid, woraufhin anschließend eine basenkatalysierte Eliminierung erfolgen kann.

Neben den beschriebenen Verfahrensweisen sind eine Reihe weiterer Zugänge zu den erfindungsgemäßen Verbindungen der Formel I denkbar. So kann es z.B. in einzelnen Fällen sinnvoll sein, die unter Verfahrensweisen a) bis k) beschriebenen Reaktionen in einer anderen Reihenfolge miteinander zu kombinieren oder analog zu den beschriebenen Methoden zunächst nicht erfindungsgemäße Verbindungen herzustellen, bei denen die Reste R(1) bis R(8) eine andere als die angegebene Bedeutung haben, und die dann in der letzten Stufe durch eine einfache Umwandlung an einem der Substituenten, wie z.B. Alkylierung, Amidierung etc., in eine erfindungsgemäße Verbindung umgewandelt werden.

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepaßt werden.

Die Verbindungen der Formel I haben überraschenderweise eine starke und spezifische blockierende (schließende) Wirkung auf einen K⁺-Kanal, der durch cyclisches Adenosinmonophosphat (cAMP) geöffnet wird und sich grundlegend vom wohlbekannten K⁺(ATP)-Kanal unterscheidet, und daß dieser an Dickdarmgewebe identifizierte K⁺(cAMP)-Kanal sehr ähnlich, vielleicht sogar identisch, mit dem am Herzmuskel identifizierten I_{Ks}-Kanal ist. Für die erfindungsgemäßen Verbindungen konnte eine starke blockierende Wirkung auf den I_{Ks}-Kanal in Meerschweinchen-Cardiomyozyten wie auch auf den in Xenopus-Oozyten exprimierten I_{sK}-Kanal gezeigt werden. Infolge dieser Blockierung des K⁺(cAMP)-Kanals bzw. des I_{Ks}-Kanals entwickeln die erfindungsgemäßen Verbindungen im lebenden Organismus pharmakologische Wirkungen von hoher therapeutischer Verwertbarkeit und eignen sich in hervorragender Weise als Arzneimittelwirkstoffe für die Therapie und Prophylaxe verschiedener Krankheitsbilder.

So zeichnen sich die erfindungsgemäßen Verbindungen der Formel I als neue Wirkstoffklasse potenter Inhibitoren der stimulierten Magensäuresekretion aus. Die Verbindungen der Formel I sind somit wertvolle Arzneimittelwirkstoffe zur Therapie und Prophylaxe von Ulcera des Magens und des intestinalen Bereiches, beispielsweise des Duodenums. Sie eignen sich ebenfalls infolge ihrer starken magensaftsekretionshemmenden Wirkung als ausgezeichnete Therapeutika zur Therapie und Prophylaxe der Refluxösophagitis.

Die erfindungsgemäßen Verbindungen der Formel I zeichnen sich weiterhin durch eine antidiarrhoische Wirkung aus und sind deshalb als Arzneimittelwirkstoffe zur Therapie und Prophylaxe von Durchfallerkrankungen geeignet.

Weiterhin eignen sich die erfindungsgemäßen Verbindungen der Formel I als Arzneimittelwirkstoffe zur Therapie und Prophylaxe von Herz-Kreislauferkrankungen. Insbesondere können sie zur Therapie und Prophylaxe aller Typen von Arrhythmien verwendet werden, einschließlich atrialer, ventrikulärer und supraventrikulärer Arrhythmien; vor allem von Herzrhythmusstörungen, die durch Aktiohspotential-Verlängerung behoben werden können. Sie können speziell angewandt werden zur Therapie und Prophylaxe von atrialer Fibrillation (Vorhofflimmern) und atrialem Flattern (Vorhofflattern) sowie zur Therapie und Prophylaxe von Reentry-Arrhythmien und zur Verhinderung des plötzlichen Herztodes infolge von Kammerflimmern.

Obwohl bereits zahlreiche antiarrhythmisch wirkende Substanzen auf dem Markt sind, gibt es doch keine Verbindung, die hinsichtlich Wirksamkeit, Anwendungsbreite und Nebenwirkungsprofil wirklich zufriedenstellend ist, so daß weiterhin eine Notwendigkeit zur Entwicklung verbesserter Antiarrhythmika besteht. Die Wirkung zahlreicher bekannter Antiarrhythmika der sogenannten Klasse III beruht auf einer Erhöhung der myocardialen Refraktärzeit durch Verlängerung der Aktionspotentialdauer. Diese wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, von dem zwei Subtypen existieren, ein schnell aktivierter I_{Kr} und ein langsam aktivierter I_{Ks}. Die meisten bekannten Klasse III-Antiarrhythmika blockieren überwiegend oder ausschließlich I_{Kr} (z.B. Dofetilid; d-Sotalol). Es hat sich jedoch gezeigt, daß diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D.M. Roden; "Current Status of Class III Antiarrhythmic Drug Therapy"; Am. J. Cardiol. 72 (1993), 44B-49B). Bei höheren Herzfrequenzen bzw. Stimulation der β-Rezeptoren hingegen ist die das Aktionspotential verlängernde Wirkung der I_{Kr}-Blocker deutlich reduziert, was darauf zurückgeführt wird, daß unter diesen Bedingungen der I_{Ks} stärker zur Repolarisierung beiträgt. Aus diesen Gründen weisen die erfindungsgemäßen Substanzen, die als I_{Ks}-Blocker wirken, wesentliche Vorteile auf gegenüber den bekannten I_{Kr}-Blockern. Inzwischen wurde auch beschrieben, daß eine Korrelation zwischen I_{Ks}-Kanal-inhibitorischer Wirkung und dem Unterbinden von lebensbedrohlichen cardialen Arrhythmien besteht, wie sie beispielsweise durch β-adrenerge Hyperstimulation ausgelöst werden (z. B. T.J. Colatsky, C.H. Follmer und C.F. Starmer; "Channel Specificity in Antiarrhythmic Drug Action; Mechanism of potassium channel block and its role in suppressing and aggravating cardiac arrhythmias"; Circulation 82 (1990), 2235 - 2242; A.E. Busch, K. Malloy, W.J. Groh, M.D. Varnum, J.P. Adelman und J. Maylie; "The novel class III antiarrhythmics NE-10064 and NE-10133 inhibit I_{sK} channels in xenopus oocytes and I_{Ks} in guinea pig cardiac myocytes"; Biochem. Biophys..Res. Commun. 202 (1994), 265 - 270).

Darüber hinaus tragen die Verbindungen zu einer deutlichen Verbesserung der Herzinsuffizienz, insbesondere der Stauungsherzinsuffizienz (Congestive Heart Failure) bei, vorteilhafterweise in der Kombination mit kontraktionsfördemden (positiv inotropen) Wirkstoffen, z.B. Phosphordiesterasehemmern.

Trotz der therapeutisch nutzbaren Vorteile, die durch eine Blockade des I_{Ks} erzielt werden können, sind bisher nur sehr wenige Verbindungen beschrieben, die diesen Subtyp des "delayed rectifiers" hemmen. Die in der Entwicklung befindliche Substanz Azimilid weist zwar auch eine blockierende Wirkung auf den I_{Ks} auf, blockiert jedoch vorwiegend den I_{Kr} (Selektivität 1:10). In der WO-A-95/14470 wird die Verwendung von Benzodiazepinen als selektive Blocker des I_{Ks} beansprucht. Weitere I_{Ks}-Blocker sind beschrieben in FEBS Letters 396 (1996), 271-275: "Specific blockade of slowly activating I_{sK} channels by chromanols ..." und Pflügers Arch. - Eur. J. Physiol. 429 (1995), 517-530: "A new class of inhibitors of cAMP-mediated Cl- secretion in rabbit colon, acting by the reduction of cAMP-activated K⁺ conductance". Die Potenz der dort genannten 3-Hydroxychromanole ist jedoch geringer als die der erfindungsgemäßen Verbindungen der Formel I.

Die erfindungsgemäßen Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanal-blockierender Wirkung. Weiterhin sind Gegenstand. der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen, pharmazeutisch einwandfreien Träger- und Hilfsstoffen-enthalten. Die pharmazeutischen Zubereitungen enthalten normalerweise 0,1 bis 90 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundiagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauf-Erkrankungen vorteilhafte Kombinationspartner kommen beispielsweise andere Antiarrhythmika, so Klasse I-, Klasse II- oder Klasse III-Antiarrhythmika, in Frage, wie beispielsweise I_{Kr}-Kanalblocker, z.B. Dofetilid, oder weiterhin blutdrucksenkende Stoffe wie ACE-Inhibitoren (beispielsweise Enalapril, Captopril, Ramipril), Angiotensin-Antagonisten, K⁺-Kanalaktivatoren; sowie alpha- und beta-Rezeptorenblocker, aber auch sympathomimetische und adrenerg wirkende Verbindungen, sowie Na⁺/H⁺-Austausch-Inhibitoren, Calciumkanalantagonisten, Phosphodiesterasehemmer und andere positiv inotrop wirkende Stoffe, wie z. B. Digitalisglykoside, oder Diuretika. Weiterhin sind Kombinationen mit antibiotisch wirkenden Substanzen und mit Antiulkusmitteln vorteilhaft, beispielsweise mit H₂-Antagonisten (z. B. Ranitidin, Cimetidin, Famotidin, etc.), insbesondere bei der Anwendung zur Behandlung von Magen-Darmerkrankungen.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wäßrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I und ihre physiologisch verträglichen Salze können auch lyophilisiert werden und die erhaltenen Lyophilisate z. B. zur . Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. üblicherweise beträgt die tägliche Dosis einer Verbindung der Formel I bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

### Experimenteller Teil

### Liste der Abkürzungen

- DMA: N,N-Dimethylacetamid
- DMSO: Dimethylsulfoxid
- EE: Essigsäurethylester
- F.p.: Schmelzpunkt
- i. Vak.: im Vakuum
- LM: Lösungsmittel
- RT: Raumtemperatur
- THF: Tetrahydrofuran

### Beispiel 1: N-[6-(3-Ethoxy-propoxy)-2,2-dimethyl-chroman-4-yl]-N-ethylmethansulfonamid

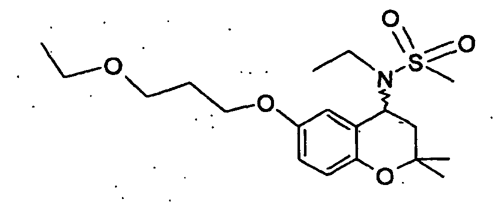

a) 2,2-Dimethyl-6-hydroxychroman-4-on
   Eine Reaktionsmischung aus 100 g (0,65 mol) 2,5-Dihydroxyacetophenon in 1 I Acetonitril, 130 ml (1,55 mol) Pyrrolidin und 290 ml (3,95 mol) Aceton wurde 8 h auf 45°C erhitzt. Dann wurden die LM i. Vak. abgezogen und der Rückstand in 1 I EE gelöst. Die organische Phase wurde 2mal mit verdünnter Salzsäure gewaschen, mit Aktivkohle verrührt und über Magnesiumsulfat getrocknet und weitgehend eingeengt. Nach Verrühren des Rückstandes mit Petrolether und Absaugen des Niederschlags wurden 102 g 2,2-Dimethyl-6-hydroxychroman-4-on, F.p. 158°C, erhalten.
b) 6-Benzyloxy-2,2-dimethylchroman-4-on
   25,2 g (131,2 mmol) 6-Hydroxy-2,2-dimethylchroman-4-on wurden bei RT unter Rühren in 350 ml Diethylketon eingetragen und nach Zugabe von 18,0 g (131 mmol) gepulvertem Kaliumcarbonat 30 min bei 75°C gerührt. Nach Abkühlen auf 60°C wurden 15,7 ml (131 mmol) Benzylbromid zugetropft, nach 2 h i.Vak. eingeengt, der Rückstand mit Wasser behandelt und der Feststoff abgesaugt, 37 g , F.p. 105 - 107 °C.
c) 6-Benzyloxy-2,2-dimethylchroman-4-onoxim
   Durch Erhitzen von 11,3 g (40 mmol) 6-Benzyloxy-2,2-dimethylchroman-4-on mit 3,1 g (44 mmol) Hydroxylaminhydrochlorid in 27 ml Ethanol und 27 ml Pyridin für 3 h auf 70°C erhielt man nach Abdestillieren des LM i. Vak. und Ausfällen mit Wasser 12,5 g Produkt, F.p. 105 -108°C. Das Produkt wurde in EE gelöst, getrocknet, eingeengt und mit Petrolether kristallisiert; F.p. 118 - 120°C.
d) 4-Amino-6-benzyloxy-2,2-dimethylchroman
   30 g 6-Benzyloxy-2,2-dimethylchroman-4-onoxim wurden in 900 ml THF/Methanol (1:1)gelöst, mit 25 ml wäßrigem Ammoniak versetzt und mit Raney - Ni in der Schüttelente hydriert. Dann wurde vom Katalysator abgesaugt, das Filtrat i. Vak. eingeengt, der Rückstand in EE gelöst, getrocknet, eingeengt und der Rückstand mit Petrolether zur Kristallisation gebracht, 22,9 g , F.p. 86 - 88°C.
e) 6-Benzyloxy-4-(methylsulfonyl)amino-2,2-dimethylchroman
   4,0 g (14 mmol) 4-Amino-6-benzyloxy-2,2-dimethylchroman wurden in 80 ml THF bei RT mit 4,2 ml (30 mmol) Triethylamin versetzt und 30 min gerührt, anschließend mit 1,95 g (1,3 ml, 17 mmol) Methansulfonsäurechlorid versetzt, wobei die Temperatur auf 40°C anstieg. Dann wurde 2 h zum Rückfluß erhitzt, über Nacht bei RT stehengelassen, i. Vak. eingeengt und der Rückstand mit Wasser behandelt; 4,9 g Produkt , F.p. 162 - 165°C.
f) N-[6-Benzyloxy-2,2-dimethyl-chroman-4-yl]-N-ethyl-methansulfonamid
   Zu einer Suspension von 0,82 g (27 mmol) Natriumhydrid (80 proz. Dispersion) in 60 ml DMA wurden bei 10°C portionsweise 7,2 g (20 mmol) 6-Benzyloxy-4-(methylsulfonyl)amino-2,2-dimethylchroman eingetragen. Nach 2h Rühren bei RT wurden 2,2 ml (26,5 mmol) Ethyljodid zugetropft, wobei die Temperatur auf 32 °C anstieg. Man erhitzte dann 2h auf 35-40°C, engte i. Vak. ein, behandelte mit Wasser, nahm das harzige Produkt mit EE auf, trocknete, engte ein und chromatographierte den Rückstand mit n-Heptan/EE (2 : 1) an Kieselgel. Aus entsprechenden Fraktionen wurden 6 g Produkt mit Petrolether/Diisopropylether (1:1) zur Kristallisation gebracht, F.p. 104 - 106°C.
g) N-[2,2-Dimethyl-6-hydroxychroman-4-yl]-N-ethyl-methansulfonamid
   12 g N-[6-Benzyloxy-2,2-dimethyl-chroman-4-yl]-N-ethyl-methansulfonamid wurden in 250 ml THF/Methanol (1:1) gelöst und mit Pd/Kohle in der Schüttelente hydriert. Nach beendeter Wasserstoffaufnahme wurde vom Katalysator abgesaugt, eingeengt und der Rückstand mit Diisopropylether zur Kristallisation gebracht, 7,7 g, F.p. 169 - 170°C.
h) 1,5 g (5 mmol) N-[2,2-Dimethyl-6-hydroxychroman-4-yl]-N-ethyl-methansulfonamid wurden in 60 ml DMA mit 1,38 g (10 mmol) gepulvertem Kaliumcarbonat 30 min auf 80°C erwärmt. Dann wurden bei 50-60°C 4 ml 3-Ethoxy-1-brompropan hinzugetropft, 2 h auf 110°C erhitzt, nach dem Abkühlen i. Vak eingeengt, der Rückstand mit Wasser und wäßriger Salzsäure behandelt, mit EE extrahiert, die organische Phase getrocknet, eingeengt und der ölige Rückstand mit n-Heptan/EE (2:1) an Kieselgel chromatographiert. Aus entsprechenden Fraktionen wurden 0,9 g N-[6-(3-Ethoxy-propoxy)-2,2-dimethyl-chroman-4-yl]-N-ethylmethansulfonamid mit Petrolether zur Kristallisation gebracht, F.p. 72 - 74°C.

### Beispiel 2

### N-Ethyl-N-[6-(5-hydroxy-pentyloxy)-2,2-dimethyl-chroman-4-yl]-methansulfonamid

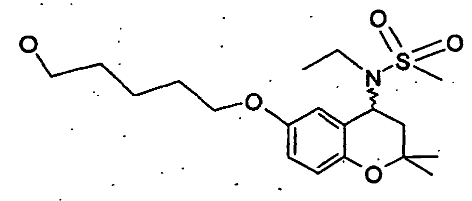

a) 4-[4-(Ethyl-methansulfonyl-amino)-2,2-dimethyl-chroman-6-yloxy]-valeriansäureethylester
   1,5 g (5 mmol)) N-[2,2-Dimethyl-6-hydroxychroman-4-yl]-N-ethyl-methansulfonamid (Beispiel 1g) wurden in 75 ml DMA mit 0,7 g gepulvertem Kaliumcarbonat 30 min bei 80°C gerührt. Dann wurden 0,8 ml (5 mmol) 5-Bromvaleriansäureethylester hinzugegeben und 120 min bei 120°C gerührt. Nach vollständiger Umsetzung (DC) wurde i.Vak. eingeengt, mit Eiswasser und wäßriger Salzsäure versetzt und kristallines Rohprodukt erhalten, das mit n-Heptan/EE (2 : 1) an Kieselgel chromatographiert wurde. Entsprechende Fraktionen wurden mit Petrolether zur Kristallisation gebracht, 1,5 g, F.p. 65 - 67°C.
b) 0,35 g (0,8 mmol) der vorstehenden Verbindung wurden in 35 ml wasserfreiem THF bei 0°C mit 1,6 ml einer 1 M Lösung von Lithiumaluminiumhydrid in THF umgesetzt (vgl. Beispiel 8).
   Nach Aufarbeitung mit EE und Einengen i. Vak. wurden 0,28 g der Titelverbindung
   mit Petrolether zur Kristallisation gebracht, F.p. 78 - 80°C.

### Beispiel 3:

### 5-[4-(Ethyl-methansulfonyl-amino)-2,2-dimethyl-chroman-6-yloxy]-pentansäure-(2-methoxy-ethyl)-amid

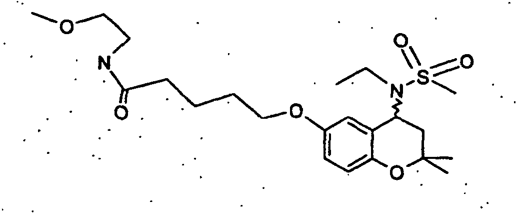

0,6 g 4-[4-(Ethyl-methansulfonyl-amino)-2,2-dimethyl-chromain-6-yloxy]-valeriansäureethylester (Beispiel 2a) wurden in 10 ml 2-Methoxyethylamin 3 Tage zum Rückfluß erhitzt. Dann wurde i. Vak. eingeengt und der Rückstand an Kieselgel chromatographiert. Mit Petrolether wurden 0,28 g der Titelverbindung zur Kristallisation gebracht, F.p. 53 - 55°C.

### Beispiel 4

### 5-[4-(Ethyl-methansulfonyl-amino)-2,2-dimethyl-chroman-6-yloxy]-pentansäure-(2-hydroxy-ethyl)-amid

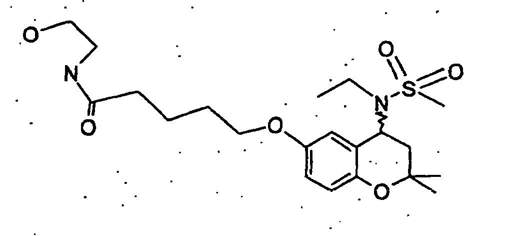

0,2 g 4-[4-(Ethyl-methansulfonyl-amino)-2,2-dimethyl-chroman-6-yloxy]-valeriansäureethylester (Beispiel 2 a) wurden in 4 ml 2-Hydroxyethylamin 1 h bei 90°C Badtemperatur erhitzt. Dann wurde mit wäßriger Salzsäure versetzt, mit EE extrahiert, getrocknet, eingeengt und der Rückstand mit Petrolether zur Kristallisation gebracht. Man erhielt 0,16 g der Titelverbindung , F.p. 88 - 90°C.

### Beispiel 5

### 4-[4-(Ethyl-methansulfonyl-amino)-2,2-dimethyl-chroman-6-yloxy]-N-(2-hydroxyethyl)-butyramid

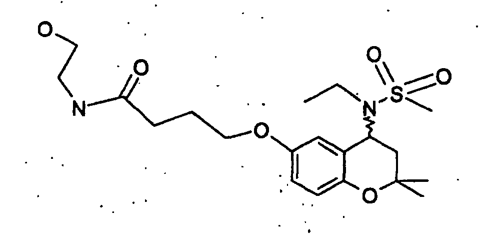

a) 4-[4-(Ethyl-methansulfonyl-amino)-2,2-dimethyl-chroman-6-yloxy]-buttersäureethylester
   1,0 g (3,3 mmol) N-[2,2-Dimethyl-6-hydroxychroman-4-yl]-N-ethylmethansulfonamid (Beispiel 1 g) wurden in 50 ml DMA mit 0,455 g gepulvertem Kaliumcarbonat 30 min bei 80-90°C gerührt. Dann wurden bei 60°C 0,71 g (0,55 ml, 3,6 mmol) 4-Brombuttersäureethylester hinzugegeben und 90 min bei 115°C gerührt. Dann wurde i.Vak. eingeengt, mit Wasser und wäßriger Salzsäure behandelt, in EE aufgenommen, getrocknet, eingeengt und der Rückstand mit n-Heptan/EE (3 : 1) an Kieselgel chromatograhiert. Entsprechende Fraktionen wurden mit Petrolether zur Kristallisation gebracht, 0,74 g, F.p. 40 - 42°C.
b) 0,2 g des vorstehenden Buttersäureesters wurden in 4 ml 2-Aminoethanol 2 h bei 95°C Badtemperatur gerührt. Dann wurde unter Kühlung mit Wasser und mit halbkonz. Salzsäure auf pH 1 gebracht, mit EE extrahiert, getrocknet, eingeengt und an der Ölpumpe getrocknet, und man erhielt 0,26 g der öligen Titelverbindung.

### Beispiel 6

### 2-[4-(Ethyl-methansulfonyl-amino)-2,2-dimethyl-chroman-6-yloxy]-N-(2-methoxyethyl)-acetamid

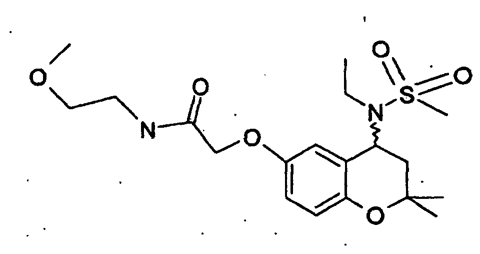

a) 4-[4-(Ethyl-methansulfonyl-amino)-2,2-dimethyl-chroman-6-yloxy]-essigsäureethylester
   1,8 g (6 mmol)) N-[2,2-Dimethyl-6-hydroxychroman-4-yl]-N-ethyl-methansulfonamid (Beispiel 1g) wurden in 80 ml DMA mit 0,83 g (6 mmol) gepulvertem Kaliumcarbonat 30 min bei 80-90°C gerührt. Dann wurden bei 60°C 0,75 ml (6,6 mmol) Bromessigsäureethylester hinzugegeben und 90 min bei 110°C gerührt. Nach Zugabe von Wasser, Salzsäure, Aufnahme in EE, Trocknen und Säulenchromatographie an Kieselgel wurden 2,4 g öliges Produkt erhalten.
b) Aus 0,24 g des vorstehenden Esters und 3 ml 2-Methoxyethylamin wurde analog Beispiel 5b die ölige Titelverbindung erhalten, 0,21 g.

### Beispiel 7

### 2-[4-(Ethyl-methansulfonyl-amino)-2,2-dimethyl-chroman-6-yloxy]-N-(2-hydroxyethyl)-acetamid

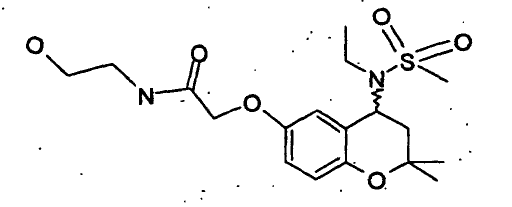

Die Titelverbindung wurde erhalten, indem 0,23 g der Verbindung aus Beispiel 6 a 1h in 3 ml 2-Aminoethanol erhitzt wurden; 0,23 g öliges Produkt.

### Beispiel 8

### N-Ethyl-N-[6-(2-hydroxy-ethoxy)-2,2-dimethyl-chroman-4-yl]-methansulfonamid

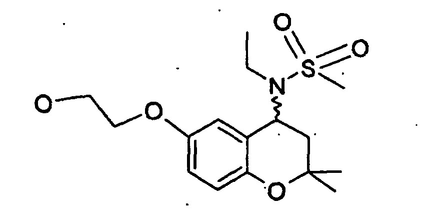

0,77 g ( 2 mmol) der Verbindung aus Beispiel 5 a wurden in 80 ml wasserfreiem THF bei 0°C unter Rühren tropfenweise mit 4 ml 1M Lösung von Lithiumaluminiumhydrid in THF versetzt. Dann wurde noch 1 h bei RT gerührt, mit Wasser und verdünnter Salzsäure versetzt, i. Vak. eingeengt, mit EE extrahiert, getrocknet, eingeengt und der Rückstand mit Diisopropylether zur Kristallisation gebracht; 0,35 g , F.p. 76 - 78°C; aus der Mutterlauge weitere 0,3 g Öl.

### Beispiel 9

### N-[6-(2-Ethoxy-ethoxy)-2,2-dimethyl-chroman-4-yl]-N-ethyl-methansulfonamid

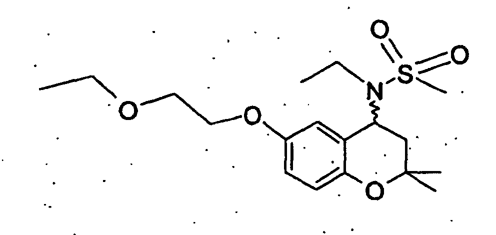

2,3 g (6,7 mmol) N-Ethyl-N-[6-(2-hydroxy-ethoxy)-2,2-dimethyl-chroman-4-yl]-methansulfonamid (Beispiel 8) wurden in 50 ml DMA unter Stickstoff mit 0,48 g (ca. 10 mmol) NaH (80proz. Dispersion) und 1,6 ml (ca. 20 mmol) Ethyljodid alkyliert. Nach Aufarbeitung und säulenchromatographischer Reinigung an Kieselgel wurden aus entsprechenden Fraktionen 1,0 g der Titelverbindung mit Petrolether zur Kristallisation gebracht, F.p. 73 - 75°C.

### Beispiel 10

### N-Ethyl-N-[6-(((4-methoxy-pyridin-2-yl)methyl)oxy)-2,2-dimethyl-chroman-4-yl]-methansulfonamid

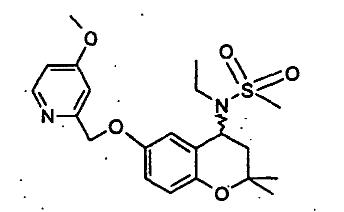

0,58 g (1,9 mmol) N-[2,2-Dimethyl-6-hydroxychroman-4-yl]-N-ethylmethansulfonamid (Beispiel 1g) wurden in 40 ml DMA mit 0,15 g (5 mmol) NaH (80%) 30 min bei 50°C gerührt. Dann tropfte man eine Lösung von 0,4 g (2 mmol) 4-Methoxy-2-chlormethylpyridin-Hydrochlorid in 5 ml DMA zu und erwärmte 2 h auf 75°C. Nach vollständiger Umsetzung wurde i.Vak. eingeengt, mit Eiswasser versetzt, mit EE extrahiert, getrocknet, eingeengt und mit Diisopropylether zur Kristallisation gebracht. Man erhielt 0,5 g, F.p. 87 - 89°C.

### Beispiel 11

### Ethansulfonsäure-[6-(2-hydroxy-ethoxy)-2,2-dimethyl-chroman-4-yl]-methyl-amid

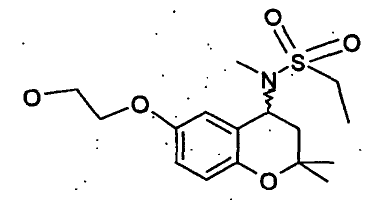

a) 6-Benzyloxy-4-(ethylsulfonyl)amino-2,2-dimethylchroman
   8,5 g (30 mmol) 4-Amino-6-benzyloxy-2,2-dimethylchroman (Beispiel 1 d) wurden in 150 ml THF unter Rühren bei RT mit 9 ml (65 mmol) Triethylamin versetzt und 30 min gerührt, anschließend mit 3,5 ml (37,5 mmol) Ethansulfonsäurechlorid versetzt, wobei die Temperatur auf 40°C ansteigt. Dann wurde 2 h bei 45°C gerührt und analog Beispiel 1e aufgearbeitet. Man erhielt 8,8 g Produkt, F.p. 145 - 149 °C (aus Wasser).
b) N-[6-Benzyloxy-2,2-dimethyl-chroman-4-yl]-N-methyl-ethansulfonamid
   Analog Beispiel 1f wurde zu einer Suspension von 1 g (25 mmol) Natriumhydrid (60 proz. Dispersion) in 75 ml DMA bei 10°C portionsweise 8,6 g (23 mmol) 6-Benzyloxy-4-(ethylsulfonyl)amino-2,2-dimethylchroman eingetragen. Nach 2 h Rühren bei RT wurden 1,6 ml (25 mmol) Methyljodid zugetropft, wobei die Temperatur auf 40 °C anstieg. Man erhitzte dann 2 h auf 35 - 40°C, engte im Vakuum ein, behandelte mit Wasser, nahm das harzige Produkt mit EE auf, trocknete, engte ein und chromatographierte den Rückstand mit n-Heptan/EE (1 : 1) an Kieselgel. Aus entsprechenden Fraktionen wurden 7,4 g Produkt mit Petrolether/Diisopropylether zur Kristallisation gebracht, F.p. 91 - 93°C.
c) N-[2,2-Dimethyl-6-hydroxychroman-4-yl]-N-methyl-ethansulfonamid
   7,2 g (18,5 mmol) N-[6-Benzyloxy-2,2-dimethyl-chroman-4-yl]-N-methylethansulfonamid wurden in 150 ml THF/Methanol (1:1) gelöst und mit Pd/Kohle in der Schüttelente hydriert. Nach beendeter Wasserstoffaufnahme wurde vom Katalysator abgesaugt, eingeengt und der Rückstand mit Diisopropylether zur Kristallisation gebracht, 5,0 g, F.p. 160 - 162°C.
d) 4-[4-(Methyl-ethansulfonyl-amino)-2,2-dimethyl-chroman-6-yloxy]-essigsäureethylester
   2,1 g (7 mmol)) N-[2,2-Dimethyl-6-hydroxychroman-4-yl]-N-methyl-ethansulfonamid wurden in DMA mit Kaliumcarbonat und 0,9 ml (8 mmol) Bromessigsäureethylester analog Beispiel 6 a umgesetzt. Man erhielt nach Behandeln mit Diisopropylether/Petrolether 2,5 g kristallines Produkt, F.p. 92 - 94°C.
e) Die Titelverbindung wurde analog Beispiel 8 aus 1,8 g des vorstehenden Esters mit Lithiumaluminiumhydrid erhalten. Aus wenig Diisopropylether kristallisierten 1,3 g Produkt, F.p. 89 - 92°C.

### Beispiel 12

### Ethansulfonsäure-[6-(2-ethoxy-ethoxy)-2,2-dimethyl-chroman-4-yl]-methyl-amid

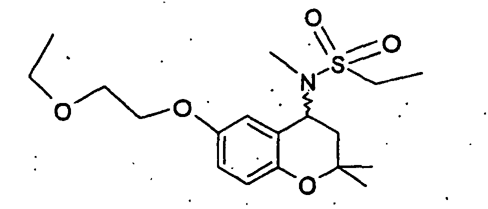

0,5 g der Verbindung aus Bsp. 11 wurden analog Bsp. 9 in DMA mit NaH und Ethyljodid umgesetzt. Nach säulenchromatographischer Reinigung an Kieselgel wurden 0,4 g der Titelverbindung mit Petrolether zur Kristallisation gebracht, F.p. 70 - 72°C.

### Beispiel 13

### 4-[4-(Ethansulfonyl-methyl-amino)-2,2-dimethyl-chroman-6-yloxymethyl]-benzoesäure-methylester

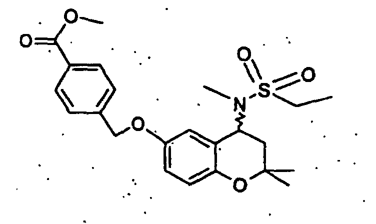

0,6 g (2 mmol) N-[2,2-Dimethyl-6-hydroxychroman-4-yl]-N-methyl-ethansulfonamid (Beispiel 11c) wurden in DMA mit gepulverter Pottasche und dann mit 0,505 g (2,2 mmol) 4-(Methoxycarbonyl)benzylbromid umgesetzt. Das Produkt wurde mit Diisopropylether zur Kristallisation gebracht, 0,72 g, F.p. 86 - 88°C.

### Beispiel 14

### 4-[4-(Ethansulfonyl-methyl-amino)-2,2-dimethyl-chroman-6-yloxymethyl]-benzoesäure

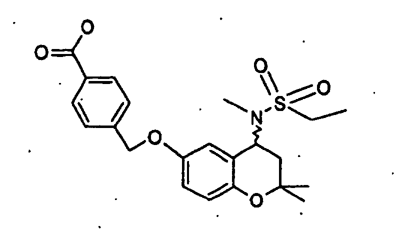

0,6 g des Esters aus Beispiel 13 wurden in 50 ml 1,5 M methanolischer NaOH 1 h bei 50°C verseift. Dann engte man i. Vak. ein, versetzte den Rückstand mit Wasser, säuerte an, gab bis zur klaren Lösung THF hinzu, saugte die Fällung ab, wusch und trocknete. Man erhielt 0,56 g der Titelverbindung, F.p. 168 - 170°C.

### Beispiel 15

### N-[6-(3-Ethoxy-propoxy)-2,2-dimethyl-chroman-4-yl]-N-methyl-methansulfonamid

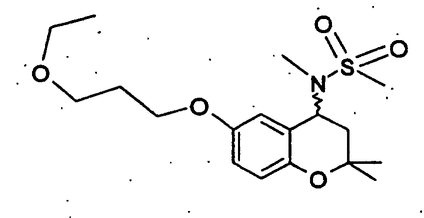

a) N-[6-Benzyloxy-2,2-dimethyl-chroman-4-yl]-N-methyl-methansulfonamid
   Zu einer Suspension von 1,2 g (30 mmol) Natriumhydrid (60 proz. Dispersion) in 75 ml DMA wurden bei 10°C portionsweise 8,9 g (25 mmol) 6-Benzyloxy-4-(methylsulfonyl)amino-2,2-dimethylchroman (Bsp. 1 e) eingetragen. Nach 2 h Rühren bei RT wurden 1,9 ml (30 mmol) Methyljodid zugetropft, wobei die Temperatur auf 50 °C anstieg. Man erhitzte dann 2 h auf 50°C, engte i. Vak. ein, behandelte mit Wasser, nahm das harzige Produkt mit EE auf, trocknete, engte ein und chromatographierte den Rückstand mit n-Heptan/EE (2 1) an Kieselgel. Aus entsprechenden Fraktionen wurden 7,4 g Produkt mit Petrolether/Diisopropylether (1:1) zur Kristallisation gebracht, F.p. 114 - 116°C.
b) N-[2,2-Dimethyl-6-hydroxychroman-4-yl]-N-methyl-methansulfonamid
   7,3 g N-[6-Benzyloxy-2,2-dimethyl-chroman-4-yl]-N-methyl-methansulfonamid wurden in 100 ml THF/Methanol (1:1) gelöst und mit Pd/Kohle in der Schüttelente hydriert. Nach beendeter Wasserstoffaufnahme wurde vom Katalysator abgesaugt, eingeengt und der Rückstand mit Diisopropylether/Petrolether zur Kristallisation gebracht, 5,2 g, F.p. 159 - 161°C.
c) 0,82 g (2,5 mmol) N-[2,2-Dimethyl-6-hydroxychroman-4-yl]-N-methylmethansulfonamid wurden in 60 ml DMA mit 0,83 g (6 mmol) gepulvertem Kaliumcarbonat 30 min auf 80°C erwärmt. Dann wurden bei 50 60°C 2 ml 3-Ethoxy-1-brompropan hinzugetropft, 3h auf 110°C erhitzt, nach dem Abkühlen i. Vak eingeengt, der Rückstand mit Wasser und wäßriger Salzsäure behandelt, mit EE extrahiert, getrocknet, eingeengt und der ölige Rückstand mit n-Heptan/EE (3 : 1) an Kieselgel chromatographiert. Aus entsprechenden Fraktionen wurden 0,74 g der Titelverbindung mit Petrolether zur Kristallisation gebracht, F.p. 61 - 63°C.

### Beispiel 16

### N-[6-(2-Hydroxy-ethoxy)-2,2-dimethyl-chroman-4-yl]-N-(2-methoxy-ethyl)-methansulfonamid

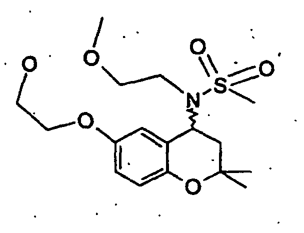

a) N-[6-Benzyloxy-2,2-dimethyl-chroman-4-yl]-N-(2-methoxyethyl)methansulfonamid Zu einer Suspension von 0,54 g (30 mmol) Natriumhydrid (60 proz. Dispersion) in 80 ml DMA wurden bei 10°C portionsweise 8,9 g (25 mmol) 6-Benzyloxy-4-(methylsulfonyl)amino-2,2-dimethylchroman (Beispiel 1e) eingetragen. Nach 30 min Rühren bei 50°C wurden bei RT 2,2 ml (22 mmol) 2-Methoxyethylbromid zugetropft. Man erhitzte dann 1 h auf 110°C, engte i. Vak. ein, behandelte mit Wasser und wäßriger Salzsäure, nahm das harzige Produkt mit EE auf, trocknete, engte ein und chromatographierte den Rückstand mit Toluol/EE (3 1) an Kieselgel. Aus entsprechenden Fraktionen wurden 2,2 g Produkt mit Petrolether zur Kristallisation gebracht, F.p. 66 - 68°C.
b) N-[2,2-Dimethyl-6-hydroxychroman-4-yl]-N-(2-methoxyethyl)-methansulfonamid 6 g N-[6-Benzyloxy-2,2-dimethyl-chroman-4-yl]-N-(2-methbxyethyl)methansulfonamid wurden in 100 ml THF/Methanol (1 : 1) gelöst und mit Pd/Kohle in der Schüttelente hydriert. Das Rohprodukt wurde mit Diisopropylether zur Kristallisation gebracht, 4,6 g, F.p. 115 - 117°C.
c) 4-[4-((2-Methoxyethyl)-methansulfonyl-amino)-2,2-dimethyl-chroman-6-yloxy]-essigsäureethylester
   2,65 g (8,0 mmol) N-[2,2-Dimethyl-6-hydroxychroman-4-yl]-N-(2-methoxyethyl)methansulfonamid wurden in 100 ml DMA mit 0,48 g (10 mmol) NaH (80%) 30 min auf 50°C erwärmt. Dann wurden analog Bsp. 6a 1,1 ml (10 mmol) Bromessigsäureethylester hinzugetropft, 1 h auf 100°C erhitzt, nach dem Abkühlen i. Vak eingeengt, der Rückstand mit Wasser und wäßriger Salzsäure behandelt, mit EE extrahiert, getrocknet, eingeengt und der ölige Rückstand mit n-Heptan/EE (1 : 1) an Kieselgel chromatographiert. Man erhielt 3,2 g öliges Produkt.
d) 2,1 g (5 mmol) des vorstehenden Esters wurden in 60 ml THF mit 10 ml 1M Lösung von Lithiumaluminiumhydrid in THF reduziert. Nach Säulenchrömatographie mit n-Heptan/EE (1 : 1) an Kieselgel wurden 1,4 g der Titelverbindung mit Diisopropylether zur Kristallisation gebracht, F.p. 61 - 63°C.

### Beispiel 17

### N-[6-(2-Ethoxy-ethoxy)-2,2-dimethyl-chroman-4-yl]-N-(2-methoxy-ethyl)-methansulfonamid

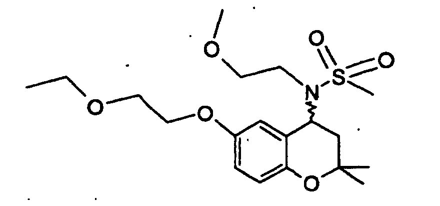

0,56 g (1,5 mmol) N-[6-(2-Hydroxy-ethoxy)-2,2-dimethyl-chroman-4-yl]-N-(2-methoxy-ethyl)- methansulfonamid (Beispiel 16) wurden analog Beispiel 9 in DMA mit NaH und Ethyljodid umgesetzt. Nach Säulenchromatographie mit n-Heptan/EE (1 1) erhielt man 0,32 g öliges Produkt.

### Beispiel 18

### 2-{4-[Methansulfonyl-(2-methoxy-ethyl)-amino]-2,2-dimethyl-chroman-6-yloxy}-N-(2-methoxy-ethyl)-acetamid

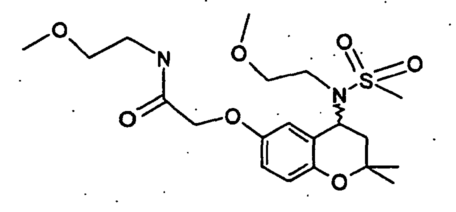

0,43 g 4-[4-((2-Methoxyethyl)-methansulfonyl-amino)-2,2-dimethyl-chroman-6-yloxy]-essigsäureethylester (Beispiel 16c) wurden in 6 ml 2-Methoxyethylamin 1 h bei 90°C erhitzt. Analog Beispiel 5b und 6b wurden 0,42 g öliges Produkt erhalten.

### Beispiel 19

### N-[6-(4-Hydroxy-butoxy)-2,2-dimethyl-chroman-4-yl]-N-(2-methoxy-ethyl)-methansulfonamid

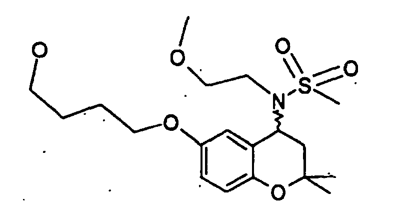

a) 4-[4-((2-Methoxyethyl)-methansulfonyl-amino)-2,2-dimethyl-chroman-6-yloxy]-buttersäureethylester
   2,0 g (6 mmol)) N-[2,2-Dimethyl-6-hydroxychroman-4-yl]-N-(2-methoxyethyl)-methansulfonamid (Beispiel 16 b) wurden analog Beispiel 5a mit Kaliumcarbonat und 4-Brombuttersäureethylester umgesetzt. Man erhielt 2,85 g öliges Produkt.
b) Die Titelverbindung wurde erhalten, indem 0,6 g (1,35 mmol) des vorstehenden Esters in THF mit 2 ml einer 1M Lösung von Lithiumaluminiumhydrid in THF reduziert wurden, 0,46 g öliges Produkt.

### Beispiel 20

### N-(2-Hydroxy-ethyl)-4-{4-[methansulfonyl-(2-methoxy-ethyl)-amino]-2,2-dimethylchroman-6-yloxy}-butyramid

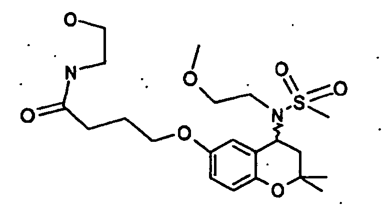

0,55 g 4-[4-((2-Methoxyethyl)-methansulfonyl-amino)-2,2-dimethyl-chroman-6-yloxy]-buttersäureethylester (Beispiel 19a) wurden in 4 ml 2-Aminoethanol 2 h bei 90°C umgesetzt, 0,6 g öliges Produkt.

### Beispiel 21

### N-[6-(3-Ethoxy-propoxy)-2,2-dimethyl-chroman-4-yl]-N-(2-methoxy-ethyl)-methansulfonamid

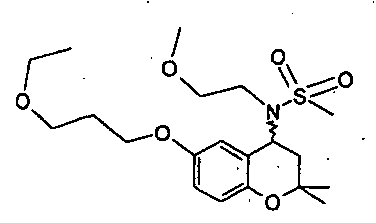

0,5 g (1,5 mmol) N-[2,2-Dimethyl-6-hydroxychroman-4-yl]-N-(2-methoxyethyl)-methansulfonamid (Beispiel 16 b) wurden analog Beispiel 1g mit Kaliumcarbonat und 3-Ethoxy-1-brompropan umgesetzt. Nach Säulenchromatographie mit n-Heptan/EE (5 : 1) an Kieselgel erhielt man 0,35 g öliges Produkt.

### Beispiel 22

### 2-[4-(Ethansulfonyl-(1-propyl)-amino)-2,2-dimethyl-chroman-6-yloxy]-N-(2-methoxyethyl)-acetamid

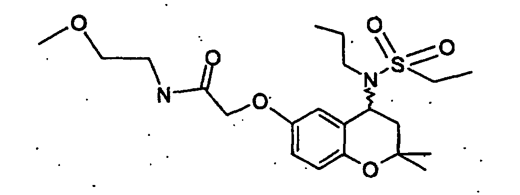

a) N-[6-Benzyloxy-2,2-dimethyl-chroman-4-yl]-N-(1-propyl)-ethansulfonamid Analog Beispiel 11 b wurde zu einer Suspension von 0,82 g (27 mmol) Natriumhydrid (80 proz. Dispersion) in 100 ml DMA bei 10°C portionsweise 7,5 g (20 mmol) 6-Benzyloxy-4-(ethylsulfonyl)amino-2,2-dimethylchroman (Beispiel 11 a) eingetragen. Nach 2 h Rühren bei RT wurden 2,4 ml (26,2 mmol) 1-Propylbromid zugetropft. Nach Aufarbeitung chromatographierte man den Rückstand mit n-Heptan/EE (1:1) an Kieselgel und aus entsprechenden Fraktionen wurden 6,0 g Produkt mit Petrolether zur Kristallisation gebracht, F.p. 102 - 103°C.
b) N-[2,2-Dimethyl-6-hydroxychroman-4-yl]-N-(1-propyl)-ethansulfonamid 6,0 g N-[6-Benzyloxy-2,2-dimethyl-chroman-4-yl]-N-(1-propyl)-ethansulfonamid wurden in 150 ml THF/Methanol (1:1) gelöst und mit Pd/Kohle in der Schüttelente hydriert, 4,4 g Produkt, F.p. 173 - 175°C (aus Petrolether).
c) 4-[4-((1-Propyl)-ethansulfonyl-amino)-2,2-dimethyl-chroman-6-yloxy]-essigsäureethylester
   0,72 g (2,2 mmol) N-[2,2-Dimethyl-6-hydroxychroman-4-yl]-N-methylethansulfonamid wurden analog Beispiel 6a mit Kaliumcarbonat und Bromessigsäureethylester umgesetzt, 0,74 g Produkt, F.p. 62 - 64°C (aus Petrolether/Diisopropylether).
d) Die Titelverbindung wurde aus 0,15 g des vorstehenden Esters mit 4 ml 2-Methoxyethylamin (1 h bei 90°C) erhalten (analog Beispiel 6b), 0,13 g Produkt, F.p. 94 - 95°C (wäßrige Salzsäure).

### Beispiel 23

### 2-[4-(Ethansulfonyl-(1-propyl)-amino)-2,2-dimethyl-chroman-6-yloxy]-N-(3-ethoxypropyl)-acetamid

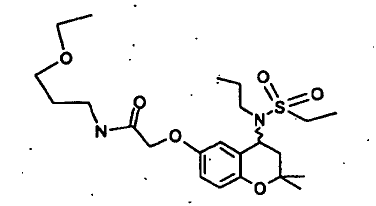

0,15 g 4-[4-((1-Propyl)-ethansulfonyl-amino)-2,2-dimethyl-chroman-6-yloxy]-essigsäureethylester wurden in 4 ml 3-Ethoxy-1-propylamin (1h 95°C) umgesetzt, analog Beispiel 5 b) und 6 b), 0,16 g öliges Produkt.

### Beispiel 24

### 2-[4-(Ethansulfonyl-(1-propyl)-amino)-2,2-dimethyl-chroman-6-yloxy]-N-(2-piperidin-1-yl- ethyl)-acetamid

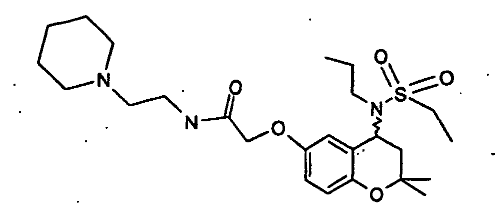

0,19 g (0,45 mmol) 4-[4-((1-Propyl)-ethansulfonyl-amino)-2,2-dimethyl-chroman-6-yloxy]-essigsäureethylester (Beispiel 22c) wurden in 3 ml N-(2-ethylamino)piperidin (2h 100°C) umgesetzt. Man erhielt 0,22g der Titelverbindung als harziges Produkt.

### Beispiel 25

### Ethansulfonsäure-[6-(2-methoxy-ethoxy)-2,2-dimethyl-chroman-4-yl]-(1-propyl)-amid

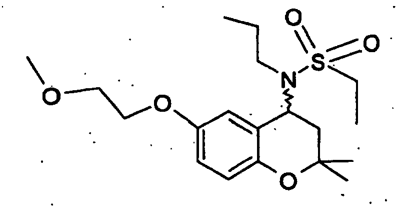

0,491 g (1,5 mmol) N-[2,2-Dimethyl-6-hydroxychroman-4-yl]-N-(1-propyl)-ethansulfonamid (Beispiel 22b) wurden in DMA mit NaH und 2-Methoxyethylbromid umgesetzt. Man erhielt 0,27 g der Titelverbindung aus Petrolether, F.p: 78 - 80°C.

### Beispiel 26

### Ethansulfonsäure-(2,2-dimethyl-6-thiophen-2-yl-chroman-4-yl)-methyl-amid

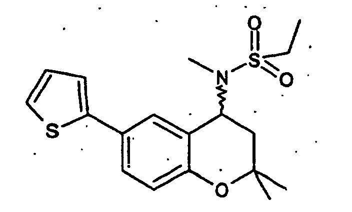

a) 2,83 g (10 mmol) Ethansulfonsäure-(2,2-dimethyl-chroman-4-yl)-methyl-amid (hergestellt aus 2-Hydroxyacetophenon analog Beispiel 1a, c, d zu 4-Amino-2,2-dimethylchroman und dann analog Beispiel 11 a, b) wurden in 16 ml Essigsäure gelöst und tropfenweise mit einer Lösung von 1,62 g (10 mmol) Iodchlorid in 16 ml Essigsäure versetzt. Nach 4 h bei RT wurde die Mischung eingeengt und mit Methylenchlorid versetzt. Man wusch mit Natriumhydrogencarbonat-Lösung neutral, trocknete und entfernte das LM im Vakuum. Das erhaltene Öl wurde säulenchromatographisch (Laufmittel Heptan/EE 1 : 1) gereinigt, und man erhielt 1,4 g (34%) Ethansulfonsäure-(6-iod-2,2-dimethyl-chroman-4-yl)-methyl-amid als Feststoff (Fp. 87 - 92°C).
b) 410 mg (1 mmol) Ethansulfonsäure-(6-iod-2,2-dimethyl-chroman-4-yl)-methylamid wurden in 5 ml Toluol gelöst und 130 mg (1 mmol) Thiophenboronsäure, 40 mg Palladium-tetrakis(triphenylphosphin) in 5 ml Ethanol und 1,1 ml 2molare Cäsiumcarbonat-Lösung zugegeben. Es wurde über Nacht bei 80°C gerührt. Nach Entfernen der LM wurde in Methylenchlorid aufgenommen und mit Wasser gewaschen. Nach Trocknen und Entfernen des LM wurde der erhaltene Rückstand säulenchromatographisch (Laufmittel Heptan/Ethylacetat 1 : 1) gereinigt, und man erhielt 260 mg (71%) Ethansulfonsäure-(2,2-dimethyl-6-thiophen-2-yl-chroman-4-yl)-methyl-amid als Öl.

### Beispiel 27: N-[6-(3-Diethylamino-propoxy)-2,2-dimethyl-chroman-4-yl]-N-methylmethansulfonamid

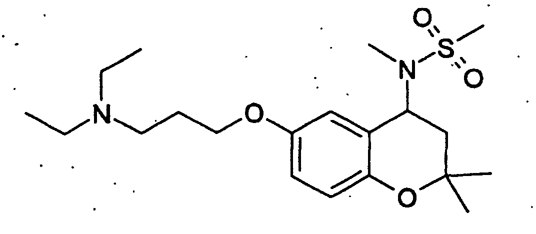

0,7 g (2,5 mmol) N-[2,2-Dimethyl-6-hydroxychroman-4-yl]-N-methylmethansulfonamid (Beispiel 15 b) wurden in 2,5 ml DMF mit 1,6 g (5,2 mmol) Phosphazenbase [tert.-Butylimino-tri(pyrrolidino)phosphoran] 1 h bei RT gerührt. Dann wurden 0,48 g (2,6 mmol) Diethylaminopropylchlorid-hydrochlocid zugegeben und es wurde 8 h auf 100°C erhitzt. Nach Abziehen des Lösungsmittels im Vakuum wurde der Rückstand in EE aufgenommen und mit Wasser gewaschen. Nach Trocknen über Magnesiumsulfat und Chromatographie über eine kurze Kieselgelsäule wurden 0,48 g N-[6-(3-Diethylamino-propoxy)-2,2-dimethyl-chroman-4-yl]-N-methyl-methansulfonamid als glasartiger Feststoff erhalten.

### Pharmakologische Untersuchungen

I_{sK}-Kanäle aus Mensch, Ratte oder Meerschweinchen wurden in Xenopus-Oozyten exprimiert. Hierfür wurden zuerst Oozyten aus Xenopus Laevis isoliert und defollikuliert. Anschließend wurde in diese Oozyten in vitro synthetisierte I_{sK}-kodierende RNA injiziert. Nach 2 - 8 Tagen I_{sK}-Proteinexpression wurden an den Oozyten mit der Zwei-Mikroelektroden Voltage-Clamp Technik I_{sK}-Ströme gemessen. Die I_{sK}-Kanäle wurden hierbei in der Regel mit 15 s dauernden Spannungssprüngen auf -10 mV aktiviert. Das Bad wurde mit einer Lösung der nachfolgenden Zusammensetzung durchspült: NaCl 96 mM, KCl 2 mM, CaCl₂ 1,8 mM, MgCl₂ 1 mM, HEPES 5 mM (titriert mit NaOH auf pH 7,5). Diese Experimente wurden bei Raumtemperatur durchgeführt. Zur Datenerhebung und Analyse wurden eingesetzt:
Geneclamp Verstärker (Axon Instruments, Foster City, USA) und MacLab D/A-Umwandler und Software (ADInstruments, Castle Hill, Australia). Die erfindungsgemäßen Substanzen wurden getestet, indem sie in unterschiedlichen Konzentrationen der Badlösung zugefügt wurden. Die Effekte der Substanzen wurden als prozentuale Inhibition des I_{sK}-Kontrollstromes berechnet, der erhalten wurde, wenn der Lösung keine Substanz zugesetzt wurde. Die Daten wurden anschließend mit der Hill-Gleichung extrapoliert, um die Hemmkonzentrationen IC₅₀ für die jeweiligen Substanzen zu bestimmen.

### Literatur:

A.E. Busch, H.-G. Kopp, S. Waldegger, I. Samarzija, H. Süßbrich, G. Raber, K. Kunzelmann, J. P. Ruppersberg und F. Lang; "Inhibition of both exogenously expressed I_{sK} and endogenous K⁺ channels in Xenopus oocytes by isosorbide dinitrate"; J. Physiol. 491 (1995), 735-741;
T. Takumi, H. Ohkubo und S. Nakanishi; "Cloning of a membrane protein that induces a slow voltage-gated potassium current"; Science 242 (1989), 1042-1045;
M.D. Varnum, A.E. Busch, C.T. Bond, J. Maylie und J.P. Adelman; "The minK channel underlies the cardiac potassium current and mediates species-specific responses to protein kinase"; C. Proc. Natl. Acad. Sci. USA 90 (1993), 11528-11532.

Auf die beschriebene Weise unter Verwendung des humanen I_{sK}-Proteins wurden für die erfindungsgemäßen Verbindungen folgende IC₅₀-Werte bestimmt:

| Verbindung | IC-50 [µM] |
|---|---|
| Beispiel 1 | 0,43 |
| Beispiel 2 | 1,71 |
| Beispiel 3 | 6,36 |
| Beispiel 8 | 7,43 |
| Beispiel 9 | 0,69 |
| Beispiel 10 | ∼ 8 |
| Beispiel 15 | 0,32 |
| Beispiel 17 | ∼ 1 |
| Beispiel 21 | 1,74 |
| Beispiel 22 | ∼ 3 |
| Beispiel 23 | 2,49 |
| Beispiel 26 | 0,38 |

## Patentansprüche

1. Verbindungen der Formel I, 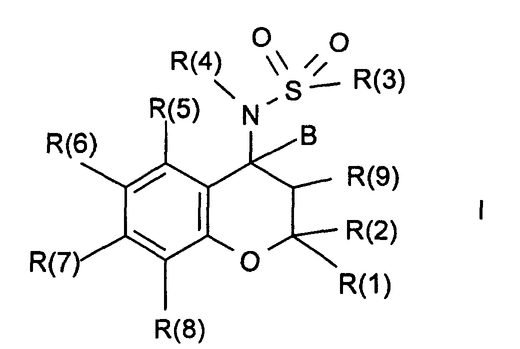 worin bedeuten:
R(1) und R(2) unabhängig voneinander Wasserstoff, CF₃, C₂F₅, C₃F₇, Alkyl mit 1, 2, 3, 4, 5 oder 6 C-Atomen oder Phenyl,
das unsubstituiert ist oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(1) und R(2) gemeinsam eine Alkylenkette mit 2, 3, 4, 5, 6, 7, 8, 9 oder 10 C-Atomen;
R(3)
R(10) -CₙH₂ₙ-NR(11)- oder R(10)-CₙH₂ₙ-,
wobei eine CH₂-Gruppe in den Gruppen CₙH₂ₙ ersetzt sein kann durch -O-, -CO-, -S-, -SO-, -SO₂- oder -NR(12a)-;
R(12a) Wasserstoff, Methyl oder Ethyl;
R(10) Wasserstoff, Methyl, Cycloalkyl mit 3, 4, 5, 6, 7 oder 8 C-Atomen, CF₃, C₂F₅ oder C₃F₇;
n Null, 1, 2, 3, 4, 5, 6, 7, 8, 9 oder 10;
R(11) Wasserstoff oder Alkyl mit 1, 2, 3, 4, 5 oder 6 C-Atomen;
oder
R(10) und R(11) gemeinsam eine Bindung, sofern n nicht kleiner als 3 ist;
oder
R(3) gemeinsam mit R(4)
eine Alkylenkette mit 3, 4, 5, 6, 7 oder 8 C-Atomen,
wobei eine CH₂-Gruppe der Alkylenkette durch -O-, -CO-, -S-, -SO-, -SO₂- oder -NR(12a)- ersetzt sein kann;
R(12a) Wasserstoff, Methyl oder Ethyl;
R(4) R(13)-CᵣH₂ᵣ,
wobei eine CH₂-Gruppe der Gruppe CᵣH₂ᵣ ersetzt sein kann durch -O-, -CH=CH-, -C≡C-, -CO-, -CO-O-, -O-CO-, -S-, -SO-, -SO₂-, - NR(14)- oder -CONR(14)-;
R(14) Wasserstoff, Alkyl mit 1, 2 oder 3 C-Atomen, - C_{y}H_{2y}-OR(12b), -C_{y}H_{2y}-NR(12b)₂;
R(12b) Wasserstoff, Methyl oder Ethyl;
y 2 oder 3;
R(13) H, CF₃, C₂F₅, C₃F₇, Cycloalkyl mit 3, 4, 5, 6, 7 oder 8 C-Atomen, -NR(15)R(16), -CONR(15)R(16), -OR(17), -COOR(17), Phenyl oder Pyrrolyl, Imidazolyl, Chinolyl, Pyrazolyl, Pyridyl, Pyrazinyl, Pyrimidinyl oder Pyridazinyl,
wobei Phenyl und der Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(15) und R(16) unabhängig voneinander Wasserstoff oder Alkyl mit 1, 2, 3 oder 4 C-Atomen;
oder
R(15) und R(16) gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen, - CₓH₂ₓOR(12c);
R(12c) Wasserstoff, Methyl oder Ethyl;
x 2 oder 3;
r Null, 1, 2, 3, 4, 5, 6, 7, 8, 9, 10, 11, 12, 13, 14, 15, 16, 17, 18, 19 oder 20;
R(6) -Y-CₛH₂ₛ-R(18), Thienyl, Furyl oder Pyrrolyl, Imidazolyl, Chinolyl, Pyrazolyl, Pyridyl, Pyrazinyl, Pyrimidinyl oder Pyridazinyl,
wobei der Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Methylamino, Dimethylamino, Ethylamino, Diethylamino, Sulfamoyl, Methylsulfonyl und Methylsulfonylamino;
Y -O-, -CO-, -O-CO-, -S-, -SO-, -SO₂-, -SO₂-O-, -SO₂NR(12d)-, - NR(12d)- oder -CONR(12d)-,
wobei die Anknüpfung an den Benzolkern jeweils über das links stehende Atom erfolgt;
R(12d) Wasserstoff, Methyl oder Ethyl;
s 1, 2, 3, 4, 5 oder 6;
R(18) substituiertes Phenyl, das einen oder zwei Substituenten trägt ausgewählt aus der Gruppe bestehend aus NO₂, CN, NH₂, N(Methyl)₂, OH, Ethyl, -COOH, -COOMethyl, -COOEthyl, -CONH₂, -CON(Methyl)₂ ;
oder
R(18) Pyrrolyl, Imidazolyl, Chinolyl, Pyrazolyl, Pyridyl, Pyrazinyl, Pyrimidinyl oder Pyridazinyl, das einen oder 2 Substituenten trägt ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
oder
R(18) -OR(19), -SO₂R(19), -NR(19)R(20), -CONR(19)R(20);
R(19) und R(20) unabhängig voneinander CₜH₂ₜ-R(21);
t Null, 1, 2, 3, 4, 5 oder 6;
R(21) Wasserstoff, CF₃, C₂F₅, C₃F₇, Cycloalkyl mit 3, 4, 5, 6, 7 oder 8 C-Atomen, NR(22)R(23), -OR(24), Phenyl, Thienyl, Pyrrolyl, Imidazolyl, Chinolyl, Pyrazolyl, Pyridyl, Pyrazinyl, Pyrimidinyl oder Pyridazinyl
wobei Phenyl, Thienyl und der Heterocyclus unsubstituiert sind oder substituiert mit 1 oder 2 Substituenten, ausgewählt aus der Gruppe bestehend aus F, Cl, Br, I, CF₃, NO₂, CN, NH₂, OH, Methyl, Ethyl, Methoxy, Dimethylamino, Sulfamoyl, Methylsulfonyl und Methylsulfonylamino;
R(22) und R(23) unabhängig voneinander Wasserstoff, Alkyl mit 1, 2 oder 3 C-Atomen;
oder
R(22) und R(23) gemeinsam eine Kette von 4 oder 5 Methylengruppen, von denen eine CH₂-Gruppe durch -O-, -S-, -NH-, -N(CH₃)- oder - N(Benzyl)- ersetzt sein kann;
R(24) Wasserstoff, Alkyl mit 1, 2 oder 3 C-Atomen;
R(5), R(7) und R(8) unabhängig voneinander Wasserstoff, F, Cl, Br, I, Alkyl mit 1, 2, 3, 4 oder 5 C-Atomen, Cycloalkyl mit 3, 4, 5, 6, 7 oder 8 C-Atomen, CN, CF₃, NO₂, OR(12e) oder NR(12e)R(12f);
R(12e) und R(12f) unabhängig voneinander Wasserstoff oder Alkyl mit 1, 2, 3 oder 4 C-Atomen;
R(9) Wasserstoff, OR(12g) oder OCOR(12g);
R(12g) Wasserstoff oder Alkyl mit 1, 2 oder 3 C-Atomen;
B Wasserstoff;
oder
R(9) und B gemeinsam eine Bindung;
sowie ihre physiologisch verträglichen Salze.

2. Verbindungen der Formel I nach Anspruch 1, worin bedeuten:
R(1) und R(2) unabhängig voneinander Wasserstoff, CF₃ oder Alkyl mit 1, 2, 3, 4, 5 oder 6 C-Atomen;
oder
R(1) und R(2) gemeinsam eine Alkylenkette mit 2, 3, 4, 5 oder 6 C-Atomen;
R(3) R(10)-CₙH₂ₙ-;
R(10) Methyl, CF₃ oder C₂F₅;
n Null, 1 oder 2;
R(4) R(13)-CᵣH₂ᵣ,
wobei eine CH₂-Gruppe der Gruppe CᵣH₂ᵣ ersetzt sein kann durch - O-, -CH=CH-, -C≡C-, -CO-, -CO-O-, -O-CO-, -S-, -SO-, -SO₂-, - NR(14)- oder -CONR(14)-;
R(14) Wasserstoff, Alkyl mit 1, 2 oder 3 C-Atomen, -C_{y}H_{2y}-OR(12b), -C_{y}H_{2y}-NR(12b)₂;
R(12b) Wasserstoff, Methyl oder Ethyl;
y 2 oder 3;
R(13) H, CF₃, C₂F₅, Cycloalkyl mit 3, 4, 5, 6 oder 7 C-Atomen, - NR(15)R(16), -CONR(15)R(16), -OR(17), -COOR(17), Phenyl oder Pyrrolyl, Imidazolyl, Chinolyl, Pyrazolyl, Pyridyl, Pyrazinyl, Pyrimidinyl oder Pyridazinyl,
wobei Phenyl und der Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
R(15) und R(16) unabhängig voneinander Wasserstoff oder Alkyl mit 1, 2, 3 oder 4 C-Atomen;
oder
R(15) und R(16) gemeinsam eine Kette von 4 oder 5 Methylengruppen, von denen eine CH₂-Gruppe durch -O-, -S-, -NH-, -N(CH₃)- oder -N(Benzyl)- ersetzt sein kann;
R(17) Wasserstoff, Alkyl mit 1, 2 oder 3 C-Atomen, - CₓH₂ₓOR(12c);
R(12c) Wasserstoff, Methyl oder Ethyl;
x 2 oder 3;
r 1, 2, 3, 4, 5, 6, 7, 8, 9, 10, 11 oder 12;
R(6) -Y-CₛH₂ₛ-P(18), Thienyl, Furyl oder Pyrrolyl, Imidazolyl, Chinotyl, Pyrazolyl, Pyridyl, Pyrazinyl, Pyrimidinyl oder Pyridazinyl
wobei Thienyl, Furyl und der Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Methylamino, Dimethylamino, Ethylamino, Diethylamino, Sulfamoyl, Methylsulfonyl und Methylsulfonylamino;
Y -O-, -CO-, -O-CO-, -S-, -SO-, -SO₂-, -SO₂-O-, -SO₂NR(12d)-, - NR(12d)- oder -CONR(12d)-,
wobei die Anknüpfung an den Benzolkern jeweils über das links stehende Atom erfolgt;
R(12d) Wasserstoff, Methyl oder Ethyl;
s 1, 2, 3, 4, 5 oder 6;
R(18) substituiertes Phenyl, das einen oder zwei Substituenten trägt ausgewählt aus der Gruppe bestehend aus NH₂, N(Methyl)₂, OH, Ethyl, -COOMethyl, -COOEthyl, -CONH₂, -CON(Methyl)₂ ;
oder
R(18) Pyrrolyl, Imidazolyl, Chinolyl, Pyrazolyl, Pyridyl, Pyrazinyl, Pyrimidinyl oder Pyridazinyl, die einen oder 2 Substituenten tragen ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
oder
R(18) -OR(19), -NR(19)R(20), -CONR(19)R(20);
R(19) und R(20) unabhängig voneinander CₜH₂ₜ-R(21);
t Null, 1, 2, 3, 4, 5 oder 6;
R(21) Wasserstoff, CF₃, NR(22)R(23), -OR(24), Phenyl, Thienyl, Pyrrolyl, Imidazolyl, Chinolyl, Pyrazolyl, Pyridyl, Pyrazinyl, Pyrimidinyl oder Pyridazinyl,
wobei Phenyl, Thienyl und der Heterocyclus unsubstituiert sind oder substituiert mit 1 oder 2 Substituenten, ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
R(22) und R(23) unabhängig voneinander Wasserstoff, Alkyl mit 1, 2 oder 3 C-Atomen;
oder
R(22) und R(23) gemeinsam eine Kette von 4 oder 5 Methylengruppen, von denen eine CH₂-Gruppe durch -O-, -S-, -NH- oder -N(CH₃)-ersetzt sein kann;
R(24) Wasserstoff, Alkyl mit 1, 2 oder 3 C-Atomen;
R(5), R(7) und R(8) unabhängig voneinander Wasserstoff, F, Cl, Br, Alkyl mit 1, 2, 3, 4 oder 5 C-Atomen, CN, CF₃, NO₂, OR(12e);
R(12e) Alkyl mit 1, 2, 3 oder 4 C-Atomen;
R(9) Wasserstoff oder OH;
B Wasserstoff;
oder
R(9) und B gemeinsam eine Bindung;
sowie ihre physiologisch verträglichen Salze.

3. Verbindungen der Formel I nach Ansprüchen 1 bis 2, worin bedeuten:
R(1) und R(2) unabhängig voneinander Wasserstoff, CF₃ oder Alkyl mit 1 oder 2 C-Atomen;
oder
R(1) und R(2) gemeinsam eine Alkylenkette mit 2, 3, 4 oder 5 C-Atomen;
R(3) Methyl oder Ethyl;
R(4) R(13)-CᵣH₂ᵣ,
wobei eine CH₂-Gruppe der Gruppe CᵣH₂ᵣ ersetzt sein kann durch -O-, -CO-O-, -O-CO-, -NR(14)- oder -CONR(14)-;
R(14) Wasserstoff oder Alkyl mit 1 oder 2 C-Atomen;
R(13) Wasserstoff, CF₃, -NR(15)R(16), -CONR(15)R(16), -OR(17), - COOR(17), Phenyl, Pyrrolyl, Imidazolyl, Chinolyl, Pyrazolyl, Pyridyl, Pyrazinyl, Pyrimidinyl oder Pyridazinyl,
wobei Phenyl und der Heterocyclus unsubstituiert sind oder substituiert mit 1 oder 2 Substituenten, ausgewählt aus der Gruppe bestehend aus F, Cl, Br, CF₃, Methyl, Methoxy, Sulfamoyl, Methylsulfonyl und Methylsulfonylamino;
R(15) und R(16) unabhängig voneinander Wasserstoff oder Alkyl mit 1 oder 2 C-Atomen;
oder
R(15) und R(16) gemeinsam eine Kette von 4 oder 5 Methylengruppen, von denen eine CH₂-Gruppe durch -O-, -NH- oder -N(CH₃)-ersetzt sein kann;
R(17) Wasserstoff oder Alkyl mit 1 oder 2 C-Atomen;
r 1, 2, 3, 4, 5, 6 oder 7;
R(6) -Y-CₛH₂ₛ-R(18), Thienyl, Furyl, Pyrrolyl, Imidazolyl, Chinolyl, Pyrazolyl, Pyridyl, Pyrazinyl, Pyrimidinyl oder Pyridazinyl
wobei Thienyl, Furyl und der Heterocyclus unsubstituiert sind oder substituiert mit 1 oder 2 Substituenten, ausgewählt aus der Gruppe bestehend aus F, Cl, Br, CF₃, Methyl, Methoxy, Methylamino, Dimethylamino, Ethylamino, Diethylamino, Sulfamoyl, Methylsulfonyl und Methylsulfonylamino;
Y -O- oder -CONR(12d)-,
wobei die Anknüpfung an den Benzolkern über das links stehende Atom erfolgt;
R(12d) Wasserstoff, Methyl oder Ethyl;
s 1, 2, 3, 4, 5 oder 6;
R(18) substituiertes Phenyl, das einen oder zwei Substituenten trägt ausgewählt aus der Gruppe bestehend aus NH₂, N(Methyl)₂, OH, -COOMethyl, -COOEthyl, -CON(Methyl)₂ ;
oder
R(18) Pyrrolyl, Imidazolyl, Chinolyl, Pyrazolyl, Pyridyl, Pyrazinyl, Pyrimidinyl oder Pyridazinyl, das einen oder 2 Substituenten trägt ausgewählt aus der Gruppe bestehend aus F, Cl, Br, CF₃, NO₂, CN, NH₂, OH, Methyl, Ethyl, Methoxy, Dimethylamino, Sulfamoyl, Methylsulfonyl und Methylsulfonylamino;
oder
R(18) -OR(19) oder -CONR(19)R(20);
R(19) und R(20) unabhängig voneinander CₜH₂ₜ-R(21);
t Null, 1, 2 oder 3;
R(21) Wasserstoff, CF₃, NR(22)R(23), -OR(24);
R(22) und R(23) unabhängig voneinander Wasserstoff, Alkyl mit 1, 2 oder 3 C-Atomen;
oder
R(22) und R(23) gemeinsam eine Kette von 4 oder 5 Methylengruppen, von denen eine CH₂-Gruppe durch -O-, -S-, -NH- oder -N(CH₃)-ersetzt sein kann;
R(24) Wasserstoff, Alkyl mit 1 oder 2 C-Atomen;
R(5), R(7) und R(8) Wasserstoff;
R(9) Wasserstoff oder OH;
B Wasserstoff;
oder
R(9) und B gemeinsam eine Bindung;
sowie ihre physiologisch verträglichen Salze.

4. Verbindungen der Formel I nach Ansprüchen 1 bis 3, worin bedeuten:
R(1) und R(2) Methyl;
R(3) Methyl oder Ethyl;
R(4) R(13)-CᵣH₂ᵣ,
wobei eine CH₂-Gruppe der Gruppe CᵣH₂ᵣ ersetzt sein kann durch -O-;
R(13) Wasserstoff, CF₃;
r 1, 2, 3, 4, 5 oder 6;
R(6) -Y-CₛH₂ₛ-R(18), Thienyl, Pyrrolyl, Imidazolyl, Chinolyl, Pyrazolyl, Pyridyl, Pyrazinyl, Pyrimidinyl oder Pyridazinyl,
wobei Thienyl und der Heterocyclus unsubstituiert sind oder substituiert mit 1 oder 2 Substituenten, ausgewählt aus der Gruppe bestehend aus F, Cl, CF₃, Methyl, Methoxy, Dimethylamino, Sulfamoyl, Methylsulfonyl und Methylsulfonylamino;
Y -O- ;
s 1, 2, 3, 4, 5 oder 6;
R(18) Pyrrolyl, Imidazolyl, Chinolyl, Pyrazolyl, Pyridyl, Pyrazinyl, Pyrimidinyl oder Pyridazinyl, das einen oder 2 Substituenten trägt ausgewählt aus der Gruppe bestehend aus F, Cl, CF₃, NO₂, CN, OH, Methyl, Methoxy, Dimethylamino, Sulfamoyl, Methylsulfonyl und Methylsulfonylamino;
oder
R(18) -OR(19) oder -CONR(19)R(20);
R(19) und R(20) unabhängig voneinander CₜH₂ₜ-R(21);
t Null, 1, 2 oder 3;
R(21) Wasserstoff, CF₃, NR(22)R(23), -OR(24);
R(22) und R(23) unabhängig voneinander Wasserstoff oder Alkyl mit 1 oder 2 C-Atomen;
R(24) Wasserstoff oder Alkyl mit 1 oder 2 C-Atomen;
R(5), R(7) und R(8) Wasserstoff;
R(9) Wasserstoff;
B Wasserstoff;

5. Verbindungen der Formel I gemäß einem oder mehreren der Ansprüche 1 bis 4 und ihre physiologisch verträglichen Salze zur Anwendung als Arzneimittel.

6. Pharmazeutische Zubereitung, enthaltend eine wirksame Menge mindestens einer Verbindung der Formel I nach einem oder mehreren der Ansprüche 1 bis 4 und/oder eines physiologisch verträglichen Salzes davon als Wirkstoff, zusammen mit pharmazeutisch annehmbaren Träger- und Zusatzstoffen und gegebenenfalls noch einem oder mehreren anderen pharmakologischen Wirkstoffen.

7. Verwendung einer Verbindung der Formel I nach einem oder mehreren der Ansprüche 1 bis 4 und/oder eines physiologisch verträglichen Salzes davon zur Herstellung eines Medikaments mit K⁺-Kanal-blockierender Wirkung zur Therapie und Prophylaxe von K⁺-Kanal mediierten Krankheiten.

8. Verwendung einer Verbindung der Formel I nach einem oder mehreren der Ansprüche 1 bis 4 und/oder eines physiologisch verträglichen Salzes davon zur Herstellung eines Medikaments zum Inhibieren der Magensäuresekretion.

9. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe von Ulcera des Magens oder des intestinalen Bereiches.

10. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe der Refluxösophagitis.

11. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe von Durchfallerkrankungen.

12. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe aller Typen von Arrhythmien, einschließlich atrialer, ventrikulärer und supraventrikulärer Arrhythmien.

13. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe von Herzrhythmusstörungen, die durch Aktionspotential-Verlängerung behoben werden können.

14. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe von atrialer Fibrillation oder atrialem Flattern.

15. Verwendung einer Verbindung der Formel I nach einem oder mehreren der Ansprüche 1 bis 4 und/oder eines physiologisch verträglichen Salzes davon zur Herstellung eines Medikaments zur Therapie oder Prophylaxe von Reentry-Arrhythmien oder zur Verhinderung des plötzlichen Herztodes infolge von Kammerflimmern.

16. Verwendung einer Verbindung der Formel I nach einem oder mehreren der Ansprüche 1 bis 4 und/oder eines physiologisch verträglichen Salzes davon zur Herstellung eines Medikaments zur Therapie der Herzinsuffizienz.

17. Verwendung einer Verbindung der Formel I nach Ansprüchen 1 bis 4 und/oder eines physiologisch verträglichen Salzes davon zum Herstellen eines Medikaments zum Inhibieren der stimulierten Magensäuresekretion, zur Therapie oder Prophylaxe von Ulcera des Magens oder des intestinalen Bereiches, der Refluxösophagitis, von Durchfallerkrankungen, zur Therapie oder Prophylaxe von Arrhythmien, einschließlich atrialer, ventrikulärer und supraventrikulärer Arrhythmien, atrialer Fibrillation und atrialem Flattern und von Reentry-Arrhythmien, oder zur Verhinderung des plötzlichen Herztodes infolge von Kammerflimmern.

## Claims

1. A compound of the formula I, 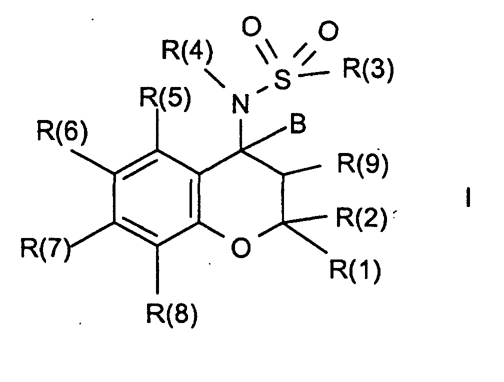 in which:
R(1) and R(2) independently of one another are hydrogen, CF₃, C₂F₅, C₃F₇, alkyl having 1, 2, 3, 4, 5 or 6 carbon atoms, or phenyl,
which is substituted or unsubstituted by 1 or 2 substituents, selected from the group consisting of F, Cl, Br, I, CF₃, NO₂ CN, NH₂, OH, methyl, ethyl, methoxy, dimethylamino, sulfamoyl, methylsulfonyl and methylsulfonylamino;
or
R(1) and R(2) together are an alkylene chain having 2, 3, 4, 5, 6, 7, 8, 9 or 10 carbon atoms;
R(3) is R(10)-CₙH₂ₙ-NR(11)- or R(10)-CₙH₂ₙ-,
where one CH₂ group in the groups CₙH₂ₙ can be replaced' by -O-, -CO-, -S-, -SO-, -SO₂- or -NR(12a)-;
R(12a) is hydrogen, methyl or ethyl;
R(10) is hydrogen, methyl, cycloalkyl having 3, 4, 5, 6, 7 or 8 carbon atoms, CF₃, C₂F₅ or C₃F₇;
n is zero, 1, 2, 3, 4, 5, 6, 7, 8, 9 or 10;
R(11) is hydrogen or alkyl having 1, 2, 3, 4, 5 or 6 carbon atoms;
or
R(10) and R(11) together are a bond, provided n is not smaller than 3;
or
R(3) together with R(4)
is an alkylene chain having 3, 4, 5, 6, 7 or 8 carbon atoms,
where one CH₂ group of the alkylene chain can be replaced by -O-, -CO-, -S-, -SO-, -SO₂- or -NR(12a)-;
R(12a) is hydrogen, methyl or ethyl;
R(4) is R(13)-CᵣH₂ᵣ,
where one CH₂ group of the group CᵣH₂ᵣ can be replaced by -O-, -CH=CH-, -C≡C-, -CO-, -CO-O-, -O-CO-, -S-, -SO-, -SO₂-, -NR(14)- or -CONR(14)-;
R(14) is hydrogen, alkyl having 1, 2 or 3 carbon atoms, -C_{y}H_{2y}-OR(12b), -C_{y}H_{2y}-NR(12b)₂;
R(12b) is hydrogen, methyl or ethyl; y is 2 or 3;
R(13) is H, CF₃, C₂F₅, C₃F₇, cycloalkyl having 3, 4, 5, 6, 7 or 8 carbon atoms, -NR(15)R(16), -CONR(15)R(16), -OR(17), -COOR(17), phenyl or pyrrolyl, imidazolyl, quinolyl, pyrazolyl, pyridyl, pyrazinyl, pyrimidinyl or pyridazinyl,
where phenyl and the heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(15) and R(16) independently of one another are hydrogen or alkyl having 1, 2, 3 or 4 carbon atoms;
or
R(15) and R(16) together are a chain of 4 or 5 methylene groups, of which one CH₂ group can be replaced by -O-, -S-, -NH-, -N(CH₃)- or -N(benzyl)-;
R(17) is hydrogen, alkyl having 1, 2 or 3 carbon atoms, -CₓH₂ₓOR(12c);
R(12c) is hydrogen, methyl or ethyl;
x is 2 or 3;
r is zero, 1, 2, 3, 4, 5, 6, 7, 8, 9, 10, 11, 12, 13, 14, 15, 16, 17, 18, 19 or 20;
R(6) is -Y-CₛH₂ₛ-R(18), thienyl, furyl or pyrrolyl, imidazolyl, quinolyl, pyrazolyl, pyridyl, pyrazinyl, pyrimidinyl or pyridazinyl,
where the heterocycle are unsubstituted or substituted by 1 or 2 substituents selected from the group consisting of F, Cl, Br, I, CF₃, NO₂, CN, NH₂, OH, methyl, ethyl, methoxy, methylamino, dimethylamino, ethylamino, diethylamino, sulfamoyl, methylsulfonyl and methylsulfonylamino;
Y is -O-, -CO-, -O-CO-, -S-, -SO-, -SO₂-, -SO₂-O-, -SO₂NR(12d)-, -NR(12d)- or -CONR(12d)-,
where the linkage to the benzene nucleus in each case takes place via the left atom;
R(12d) is hydrogen, methyl or ethyl;
s is 1, 2, 3, 4, 5 or 6;
R(18) is substituted phenyl which carries one or two substituents selected from the group consisting of NO₂, CN, NH₂, N(methyl)₂, OH, ethyl, -COOH, -COOmethyl, -COOethyl, -CONH₂, -CON(methyl)₂;
or
R(18) is pyrrolyl, imidazolyl, quinolyl, pyrazolyl, pyridyl, pyrazinyl, pyrimidinyl or pyridazinyl, which carries one or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
or
R(18) is -OR(19), -SO₂R(19), -NR(19)R(20), -CONR(19)R(20);
R(19) and R(20) independently of one another are CₜH₂ₜ-R(21);
t is zero, 1, 2, 3, 4, 5 or 6;
R(21) is hydrogen, CF₃, C₂F₅, C₃F₇, cycloalkyl having 3, 4, 5, 6, 7 or 8 carbon atoms, NR(22)R(23), -OR(24), phenyl, thienyl, pyrrolyl, imidazolyl, quinolyl, pyrazolyl, pyridyl, pyrazinyl, pyrimidinyl or pyridazinyl,
where phenyl, thienyl and the heterocycle are unsubstituted or substituted by 1 or 2 substituents selected from the group consisting of F, Cl, Br, I, CF₃, NO₂, CN, NH₂, OH, methyl, ethyl, methoxy, dimethylamino, sulfamoyl, methylsulfonyl and methylsulfonylamino;
R(22) and R(23) independently of one another are hydrogen, alkyl having 1, 2 or 3 carbon atoms;
or
R(22) and R(23) together are a chain of 4 or 5 methylene groups, of which one CH₂ group can be replaced by -O-, -S-, -NH-, -N(CH₃)- or -N(benzyl)-;
R(24) is hydrogen, alkyl having 1, 2 or 3 carbon atoms;
R(5), R(7) and R(8) independently of one another are hydrogen, F, Cl, Br, I, alkyl having 1, 2, 3, 4 or 5 carbon atoms, cycloalkyl having 3, 4, 5, 6, 7 or 8 carbon atoms, CN, CF₃, NO₂, OR(12e) or NR(12e)R(12f);
R(12e) and R(12f) independently of one another are hydrogen or alkyl having 1, 2, 3 or 4 carbon atoms;
R(9) is hydrogen, OR(12g) or OCOR(12g);
R(12g) is hydrogen or alkyl having 1, 2 or 3 carbon atoms;
B is hydrogen;
or
R(9) and B together are a bond;
or its physiologically tolerable salts.

2. A compound of the formula I as claimed in claim 1, in which:
R(1) and R(2) independently of one another are hydrogen, CF₃ or alkyl having 1, 2, 3, 4, 5 or 6 carbon atoms;
or
R(1) and R(2) together are an alkylene chain having 2, 3, 4, 5 or 6 carbon atoms;
R(3) is R(10)-CₙH₂ₙ-;
R(10) is methyl, CF₃ or C₂F₅;
n is zero, 1 or 2;
R(4) is R(13)-CᵣH₂ᵣ,
where one CH₂ group of the group CᵣH₂ᵣ can be replaced by -O-, -CH=CH-, -C≡C-, -CO-, -CO-O-, -O-CO-, -S-, -SO-, -SO₂-, -NR(14)- or -CONR(14)-;
R(14) is hydrogen, alkyl having 1, 2 or 3 carbon atoms, -C_{y}H_{2y}-OR(12b), -C_{y}H_{2y}-NR(12b)₂;
R(12b) is hydrogen, methyl or ethyl;
y is 2 or 3
R(13) is H, CF₃, C₂F₅, cycloalkyl having 3, 4, 5, 6 or 7 carbon atoms, -NR(15)R(16), -CONR(15)R(16), -OR(17), -COOR(17), phenyl or pyrrolyl, imidazolyl, quinolyl, pyrazolyl, pyridyl, pyrazinyl, pyrimidinyl or pyridazinyl,
where phenyl and the heterocycle are unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
R(15) and R(16) independently of one another are hydrogen or alkyl having 1, 2, 3 or 4 carbon atoms;
or
R(15) and R(16) 'together are a chain of 4 or 5 methylene groups, of which one CH₂ group can be replaced by -O-, -S-, -NH-, -N(CH₃)- or -N(benzyl)-;
R(17) is hydrogen, alkyl having 1, 2 or 3 carbon atoms, -CₓH₂ₓOR(12c);
R(12c) is hydrogen, methyl or ethyl;
x is 2 or 3;
r is 1, 2, 3, 4, 5, 6, 7, 8, 9, 10, 11 or 12;
R(6) is -Y-CₛH₂ₛ-R(18), thienyl, furyl or pyrrolyl, imidazolyl, quinolyl, pyrazolyl, pyridyl, pyrazinyl, pyrimidinyl or pyridazinyl,
where thienyl, furyl and the heterocycle are unsubstituted or substituted by 1 or 2 substituents selected from the group consisting of F, Cl, Br, CF₃, NO₂, CN, NH₂, OH, methyl, ethyl, methoxy, methylamino, dimethylamino, ethylamino, diethylamino, sulfamoyl, methylsulfonyl and methylsulfonylamino;
Y is -O-, -CO-, -O-CO-, -S-, -SO-, -SO₂-, -SO₂-O-, -SO₂NR(12d)-, -NR(12d)- or -CONR(12d)-,
where the linkage to the benzene nucleus in each case takes place via the left atom;
R(12d) is hydrogen, methyl or ethyl;
s is 1, 2, 3, 4, 5 or 6;
R(18) is substituted phenyl which carries one or two substituents selected from the group consisting of NH₂, N(methyl)₂, OH, ethyl, -COOmethyl, -COOethyl, -CONH₂, -CON(methyl)₂;
or
R(18) is pyrrolyl, imidazolyl, quinolyl, pyrazolyl, pyridyl, pyrazinyl, pyrimidinyl or pyridazinyl, which carries one or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
or
R(18) is -OR(19), -NR(19)R(20), -CONR(19)R(20);
R(19) and R(20) independently of one another are CₜH₂ₜ-R(21);
t is zero, 1, 2, 3, 4, 5 or 6;
R(21) is hydrogen, CF₃, NR(22)R(23), -OR(24), phenyl, thienyl, pyrrolyl, imidazolyl, quinolyl, pyrazolyl, pyridyl, pyrazinyl, pyrimidinyl or pyridazinyl,
where phenyl, thienyl and the heterocycle are unsubstituted or substituted by 1 or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
R(22) and R(23) independently of one another are hydrogen, alkyl having 1, 2 or 3 carbon atoms;
or
R(22) and R(23) together are a chain of 4 or 5 methylene groups, of which one CH₂ group can be replaced by -O-, -S-, -NH- or -N(CH₃)-;
R(24) is hydrogen, alkyl having 1, 2 or 3 carbon atoms;
R(5), R(7) and R(8) independently of one another are hydrogen, F, Cl, Br, alkyl having 1, 2, 3, 4 or 5 carbon atoms, CN, CF₃, NO₂, OR(12e);
R(12e) is alkyl having 1, 2, 3 or 4 carbon atoms;
R(9) is hydrogen or OH;
B is hydrogen;
or
R(9) and B together are a bond;
or its physiologically tolerable salts.

3. A compound of the formula I as claimed in claims 1 to 2, in which:
R(1) and R(2) independently of one another are hydrogen, CF₃ or alkyl having 1 or 2 carbon atoms;
or
R(1) and R(2) together are an alkylene chain having 2, 3, 4 or 5 carbon atoms;
R(3) is methyl or ethyl;
R(4) is R(13)-CᵣH₂ᵣ,
where one CH₂ group of the group CᵣH₂ᵣ can be replaced by -O-, -CO-O-, -O-CO-, -NR(14)- or -CONR(14)-;
R(14) is hydrogen or alkyl having 1 or 2 carbon atoms;
R(13) is hydrogen, CF₃, -NR(15)R(16), -CONR(15)R(16), -OR(17), -COOR(17), phenyl, pyrrolyl, imidazolyl, quinolyl, pyrazolyl, pyridyl, pyrazinyl, pyrimidinyl or pyridazinyl,
where phenyl and the heterocycle are unsubstituted or substituted by 1 or 2 substituents selected from the group consisting of F, Cl, Br, CF₃, methyl, methoxy, sulfamoyl, methylsulfonyl and methylsulfonylamino;
R(15) and R(16) independently of one another are hydrogen or alkyl having 1 or 2 carbon atoms;
or
R(15) and R(16) together are a chain of 4 or 5 methylene groups, of which one CH₂ group can be replaced by -O-, -NH- or -N(CH₃)-;
R(17) is hydrogen or alkyl having 1 or 2 carbon atoms;
r 1, 2, 3, 4, 5, 6 or 7;
R(6) is -Y-CₛH₂ₛ-R(18), thienyl, furyl, pyrrolyl, imidazolyl, quinolyl, pyrazolyl, pyridyl, pyrazinyl, pyrimidinyl or pyridazinyl,
where thienyl, furyl and the heterocycle are unsubstituted or substituted by 1 or 2 substituents selected from the group consisting of F, Cl, Br, CF₃, methyl, methoxy, methylamino, dimethylamino, ethylamino, diethylamino, sulfamoyl, methylsulfonyl and methylsulfonylamino;
Y is -O- or -CONR(12d)-,
where the linkage to the benzene nucleus takes place via the left atom;
R(12d) is hydrogen, methyl or ethyl;
s is 1, 2, 3, 4, 5 or 6;
R(18) is substituted phenyl which carries one or two substituents selected from the group consisting of NH₂, N(methyl)₂, OH, -COOmethyl, -COOethyl, -CON(methyl)₂ ;
or
R(18) is pyrrolyl, imidazolyl, quinolyl, pyrazolyl, pyridyl, pyrazinyl, pyrimidinyl or pyridazinyl, which carries one or 2 substituents selected from the group consisting of F, Cl, Br, CF₃, NO₂, CN, NH₂, OH, methyl, ethyl, methoxy, dimethylamino, sulfamoyl, methylsulfonyl and methylsulfonylamino;
or
R(18) is-OR(19)or-CONR(19)R(20);
R(19) and R(20) independently of one another are CₜH₂ₜ-R(21);
t is zero, 1, 2 or 3;
R(21) is hydrogen, CF₃, NR(22)R(23), -OR(24);
R(22) and R(23) independently of one another are hydrogen, alkyl having 1, 2 or 3 carbon atoms;
or
R(22) and R(23) together are a chain of 4 or 5 methylene groups, of which one CH₂ group can be replaced by -O-, -S-, -NH- or -N(CH₃)-;
R(24) is hydrogen, alkyl having 1 or 2 carbon atoms;
R(5), R(7) and R(8) are hydrogen;
R(9) is hydrogen or OH;
B is hydrogen;
or
R(9) and B together are a bond;
or its physiologically tolerable salts.

4. A compound of the formula I as claimed in claims 1 to 3, in which:
R(1) and R(2) are methyl;
R(3) is methyl or ethyl;
R(4) is R(13)-CᵣH₂ᵣ,
where one CH₂ group of the group CᵣH₂ᵣ can be replaced by -O-;
R(13) is hydrogen, CF₃;
r is 1, 2, 3, 4, 5 or 6;
R(6) is -Y-CₛH₂ₛ-R(18), thienyl, pyrrolyl, imidazolyl, quinolyl, pyrazolyl, pyridyl, pyrazinyl, pyrimidinyl or pyridazinyl,
where thienyl and the heterocycle are unsubstituted or substituted by 1 or 2 substituents selected from the group consisting of F, Cl, CF₃, methyl, methoxy, dimethylamino,
sulfamoyl, methylsulfonyl and methylsulfonylamino;
Y is -O- ;
s is 1, 2, 3, 4, 5 or 6;
R(18) is pyrrolyl, imidazolyl, quinolyl, pyrazolyl, pyridyl, pyrazinyl, pyrimidinyl or pyridazinyl, which carries one or 2 substituents selected from the group consisting of F, Cl, CF₃, NO₂, CN, OH, methyl, methoxy, dimethylamino, sulfamoyl, methylsulfonyl and methylsulfonylamino;
or
R(18) is -OR(19) or -CONR(19)R(20);
R(19) and R(20) independently of one another are CₜH₂ₜ-R(21);
t is zero, 1, 2 or 3;
R(21) is hydrogen, CF₃, NR(22)R(23), -OR(24);
R(22) and R(23) independently of one another are hydrogen or alkyl having 1 or 2 carbon atoms;
R(24) is hydrogen or alkyl having 1 or 2 carbon atoms;
R(5), R(7) and R(8) are hydrogen;
R(9) is hydrogen;
B is hydrogen.

5. A compound of the formula I as claimed in one or more of claims 1 to 4 or its physiologically tolerable salts for use as a pharmaceutical.

6. A pharmaceutical preparation comprising an efficacious amount of at least one compound of the formula I as claimed in one or more of claims 1 to 4 and/or a physiologically tolerable salt thereof as active compound, together with pharmaceutically acceptable excipients and additives and, if appropriate, additionally one or more other pharmacological active compounds.

7. The use of a compound of the formula I as claimed in one or more claims 1 to 4 and/or of a physiologically tolerable salt thereof for the production of a medicament having K⁺ channel-blocking action for the therapy and prophylaxis of K⁺ channel-mediated diseases.

8. The use of a compound of the formula I as claimed in one or more of claims 1 to 4 and/or of a physiologically tolerable salt thereof for the production of a medicament for inhibiting gastric acid secretion.

9. The use of a compound of the formula I as claimed in one or more of claims 1 to 4 and/or of a physiologically tolerable salt thereof for the production of a medicament for the therapy or prophylaxis of ulcers of the stomach or of the intestinal region.

10. The use of a compound of the formula I as claimed in one or more of claims 1 to 4 and/or of a physiologically tolerable salt thereof for the production of a medicament for the therapy or prophylaxis of reflux esophagitis.

11. The use of a compound of the formula I as claimed in one or more of claims 1 to 4 and/or of a physiologically tolerable salt thereof for the production of a medicament for the therapy or prophylaxis of diarrheal disorders.

12. The use of a compound of the formula I as claimed in one or more of claims 1 to 4 and/or of a physiologically tolerable salt thereof for the production of a medicament for the therapy or prophylaxis of all types of arrhythmias, including atrial, ventricular and supraventricular arrhythmias.

13. The use of a compound of the formula I as claimed in one or more of claims 1 to 4 and/or of a physiologically tolerable salt thereof for the production of a medicament for the therapy or prophylaxis of cardiac arrhythmias which can be eliminated by action potential prolongation.

14. The use of a compound of the formula I as claimed in one or more of claims 1 to 4 and/or of a physiologically tolerable salt thereof for the production of a medicament for the therapy or prophylaxis of atrial fibrillation or atrial flutters.

15. The use of a compound of the formula I as claimed in one or more of claims 1 to 4 and/or of a physiologically tolerable salt thereof for the production of a medicament for the therapy or prophylaxis of reentry arrhythmias or for the prevention of sudden heart death as a result of ventricular fibrillation.

16. The use of a compound of the formula I as claimed in one or more of claims 1 to 4 and/or of a physiologically tolerable salt thereof for the production of a medicament for the therapy of cardiac insufficiency.

17. The use of a compound of the formula I as claimed in one or more of claims 1 to 4 and/or of a physiologically tolerable salt thereof for the production of a medicament for inhibiting stimulated gastric acid secretion, for the therapy or prophylaxis of ulcers of the stomach or of the intestinal region, of reflux esophagitis, of diarrheal disorders, for the therapy or prophylaxis of arrhythmias, including atrial, ventricular and supraventricular arrhythmias, atrial fibrillation and atrial flutters and of reentry arrhythmias, or for the prevention of sudden heart death as a result of ventricular fibrillation.

## Revendications

1. Composés de formule 1 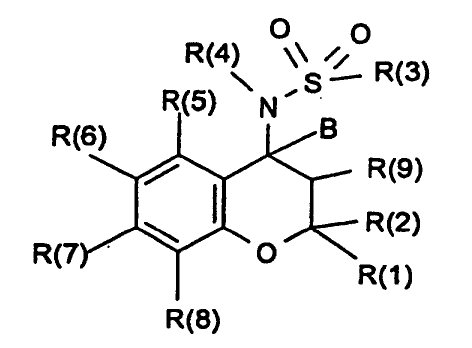 dans laquelle
R(1) et R(2) représentent, indépendamment l'un de l'autre, un atome d'hydrogène ou un reste CF₃, C₂F₅, C₃F₇ ou alkyle de 1, 2, 3, 4, 5 ou 6 atomes de carbone ou un reste phényle non substitué ou substitué par 1 ou 2 substituants choisi dans le groupe constitué par les restes F, Cl, Br, I, CF₃, NO₂, CN, NH₂, OH, méthyle, éthyle, méthoxy, diméthylamino, sulfamoyle, méthylsulfonyle et méthylsulfonylamino; ou
R(1) et R(2) forment ensemble une chaîne alkylène de 2, 3, 4, 5, 6, 7, 8, 9 ou 10 atomes de carbone;
R(3) représente un groupe R(10)-CₙH₂ₙ-NR(11)- ou R(10)-CₙH₂ₙ-, dans lesquels un groupe CH₂ des groupes CₙH₂ₙ peut être remplacé par -O-, -CO-, -S-, - SO-, -SO₂- ou -NR(12a)-;
R(10) représente un atome d'hydrogène ou un reste méthyle, cycloalkyle de 3, 4, 5, 6, 7 ou 8 atomes de carbone, CF₃, C₂F₅ ou C₃F₇;
n est 0, 1, 2, 3, 4, 5, 6, 7, 8, 9 ou 10;
R(11) représente un atome d'hydrogène ou un reste alkyle de 1, 2, 3, 4, 5 ou 6 atomes de carbone; ou
R(10) et R(11) forment ensemble une liaison, dans la mesure où n n'est pas inférieur à 3; ou
R(3) forme avec R(4) une chaîne alkylène de 3, 4, 5, 6, 7 ou 8 atomes de carbone, un groupe CH₂ de la chaîne alkylène pouvant être remplacé par -O-, -CO-, - S-, -SO-, -SO₂- ou -N(R12a)-;
R(12a) représente un atome d'hydrogène ou un reste méthyle ou éthyle;
R(4) représente un groupe R(13)-CᵣH₂ᵣ dans lequel un groupe CH₂ du groupe CᵣH₂ᵣ peut être remplacé par -O-, -CH=CH-, -C≡C-, -CO-, -CO-O-, -O-CO-, -S-, - SO-, -SO₂-, -NR(14)- ou -CONR(14)-;
R(14) représente un atome d'hydrogène, un reste alkyle de 1, 2 ou 3 atomes de carbone, -C_{y}H_{2y}-OR(12b), -C_{y}H_{2y}-NR(12b)₂;
R(12b) représente un atome d'hydrogène ou un reste méthyle ou éthyle;
y est 2 ou 3;
R(13) représente H ou un reste CF₃, C₂F₅, C₃F₇, cycloalkyle de 3, 4, 5, 6, 7 ou 8 atomes de carbone, -NR(15)R(16), -CONR(15)R(16), -OR(17), - COOR(17), phényle ou pyrrolyle, imidazolyle, quinoléyle, pyrazolyle, pyridyle, pyrazinyle, pyrimidinyle ou pyridazinyle, le reste phényle et l'hétérocycle étant non substitués ou substitués par 1 ou 2 substituants choisis dans le groupe constitué par les restes F, CI, Br, I, CF₃, NO₂, CN, NH₂, OH, méthyle, éthyle, méthoxy, diméthylamino, sulfamoyle, méthylsulfonyle et méthylsulfonylamino;
R(15) et R(16) représentent, indépendamment l'un de l'autre, un atome d'hydrogène ou un reste alkyle de 1, 2, 3 ou 4 atomes de carbone; ou
R(15) et R(16) forment ensemble une chaîne de 4 ou 5 groupes méthylène dont un groupe CH₂ peut être remplacé par -O-, -S-, - NH-, -N(CH₃)- ou -N(benzyle)-;
R(17) représente un atome d'hydrogène ou un reste alkyle de 1, 2 ou 3 atomes de carbone ou -CₓH₂ₓOR(12c);
R(12c) représente un atome d'hydrogène ou un reste méthyle ou éthyle;
x est 2 ou 3;
r est 0, 1, 2, 3, 4, 5, 6, 7, 8, 9, 10, 11, 12, 13, 14, 15, 16, 17, 18, 19 ou 20;
R(6) représente un reste -Y-CₛH₂ₛ-R(18), thiényle, furyle ou pyrrolyle, imidazolyle, quinoléyle, pyrazolyle, pyridyle, pyrazinyle, pyrimidinyle ou pyridazinyle, l'hétérocycle étant non substitué ou substitué par 1 ou 2 substituants choisis dans le groupe constitué par les restes F, Cl, Br, I, CF₃, NO₂, CN, NH₂, OH, méthyle, éthyle, méthoxy, méthylamino, diméthylamino, éthylamino, diéthylamino, sulfamoyle, méthylsulfonyle et méthylsulfonylamino;
Y représente -O-, -CO-, -O-CO-, -S-, -SO-, -SO₂-, -SO₂-O-, - SO₂NR(12d)-, -NR(12d)- ou -CONR(12d)-, la liaison au cycle benzène se faisant toujours par l'intermédiaire de l'atome de gauche;
R(12d) est un atome d'hydrogène ou un reste méthyle ou éthyle;
s est 1, 2, 3, 4, 5 ou 6;
R(18) est un reste phényle substitué portant un ou deux substituants choisis dans le groupe constitué par les restes NO₂, CN, NH₂, N(méthyle)₂, OH, éthyle, -COOH, -COOméthyle, -COOéthyle, -CONH₂, - CON(méthyle)₂; ou
R(18) représente un reste pyrrolyle, imidazolyle, quinoléyle, pyrazolyle, pyridyle, pyrazinyle, pyrimidinyle ou pyridazinyle portant un ou deux substituants choisis dans le groupe constitué par les restes F, Cl, Br, I, CF₃, NO₂, CN, NH₂, OH, méthyle, éthyle, méthoxy, diméthylamino, sulfamoyle, méthylsulfonyle et méthylsulfonylamino; ou
R(18) représente un reste -OR(19), -SO₂R(19), -NR(19)R(20), - CONR(19)R(20);
R(19) et R(20) représentent, indépendamment l'un de l'autre, un reste CₜH₂ₜ-R(21);
t est 0,1,2, 3,4,5 ou 6;
R(21) représente un atome d'hydrogène ou un reste CF₃, C₂F₅, C₃F₇, cycloalkyle de 3, 4, 5, 6, 7 ou 8 atomes de carbone, NR(22)R(23), -OR(24), phényle, thiényle, pyrrolyle, imidazolyle, quinoléyle, pyrazolyle, pyridyle, pyrazinyle, pyrimidinyle ou pyridazinyle, les restes phényle, thiényle et hétérocycliques étant non substitués ou substitués par 1 ou 2 substituants choisis dans le groupe constitué par les restes F, Cl, Br, I, CF₃, NO₂, CN, NH₂, OH, méthyle, éthyle, méthoxy, diméthylamino, sulfamoyle, méthylsulfonyle et méthylsulfonylamino;
R(22) et R(23) représentent, indépendamment l'un de l'autre, un atome d'hydrogène ou un reste alkyle de 1, 2 ou 3 atomes de carbone; ou
R(22) et R(23) forment ensemble une chaîne de 4 ou 5 groupes méthylène dont un groupe CH₂ peut être remplacé par -O-, -S-, -NH-, -N(CH₃)- ou - N(benzyle)-;
R(24) représente un atome d'hydrogène ou un reste alkyle de 1, 2 ou 3 atomes de carbone:
R(5), R(7) et R(8) représentent, indépendamment les uns des autres, un atome d'hydrogène ou un reste F, Cl, Br, I, alkyle de 1, 2, 3, 4 ou 5 atomes de carbone, cycloalkyle de 3, 4, 5, 6, 7 ou 8 atomes de carbone, CN, CF₃, NO₂, OR(12e) ou NR(12e)(12f);
R(12e) et R(12f) représentent, indépendamment l'un de l'autre, un atome d'hydrogène ou un reste alkyle de 1, 2, 3 ou 4 atomes de carbone;
R(9) représente un atome d'hydrogène ou un reste OR(12g) ou OCOR(12g); R(12g)représente un atome d'hydrogène ou un reste alkyle de 1, 2 ou 3 atomes de carbone;
B représente un atome d'hydrogène, ou
R(9) et B forment ensemble une liaison;
et leurs sels physiologiquement acceptables.

2. Composés de formule I selon la revendication 1, dans lesquels:
R(1) et R(2) représentent, indépendamment l'un de l'autre, un atome d'hydrogène ou un reste CF₃ ou alkyle de 1, 2, 3, 4, 5 ou 6 atomes de carbone; ou
R(1) et R(2) forment ensemble une chaîne alkylène de 2, 3, 4, 5 ou 6 atomes de carbone;
R(3) représente un groupe R(10)-CₙH₂ₙ-;
R(10) représente un reste méthyle, CF₃ ou C₂F₅;
n est 0, 1 ou 2;
R(4) représente un groupe R(13)-CᵣH₂ᵣ dans lequel un groupe CH₂ du groupe CᵣH₂ᵣ peut être remplacé par -O-, -CH=CH-, -C≡C-, -CO-, -CO-O-, -O-CO-, -S-, - SO-, -SO₂-, -NR(14)- ou -CONR(14)-;
R(14) représente un atome d'hydrogène, un reste alkyle de 1, 2 ou 3 atomes de carbone, -C_{y}H_{2y}-OR(12b), -C_{y}H_{2y}-NR(12b)₂;
R(12b) représente un atome d'hydrogène ou un reste méthyle ou éthyle;
y est 2 ou 3;
R(13) représente H ou un reste CF₃, C₂F₅, cycloalkyle de 3, 4, 5, 6 ou 7 atomes de carbone, -NR(15)R(16), -CONR(15)R(16), -OR(17), -COOR(17), phényle ou pyrrolyle, imidazolyle, quinoléyle, pyrazolyle, pyridyle, pyrazinyle, pyrimidinyle ou pyridazinyle, le reste phényle et l'hétérocycle étant non substitués ou substitués par 1 ou 2 substituants choisis dans le groupe constitué par les restes F, Cl, Br, CF₃, NO₂, CN, NH₂, OH, méthyle, éthyle, méthoxy, diméthylamino, sulfamoyle, méthylsulfonyle et méthylsulfonylamino;
R(15) et R(16) représentent, indépendamment l'un de l'autre, un atome d'hydrogène ou un reste alkyle de 1, 2, 3 ou 4 atomes de carbone; ou
R(15) et R(16) forment ensemble une chaîne de 4 ou 5 groupes méthylène dont un groupe CH₂ peut être remplacé par -O-, -S-, - NH-, -N(CH₃)- ou -N(benzyle)-;
R(17) représente un atome d'hydrogène ou un reste alkyle de 1, 2 ou 3 atomes de carbone ou ―CₓH₂ₓOR(12c);
R(12c) représente un atome d'hydrogène ou un reste méthyle ou éthyle;
x est 2 ou 3;
r est 1, 2, 3, 4, 5, 6, 7, 8, 9, 10, 11 ou 12;
R(6) représente un reste -Y-CₛH₂ₛ-R(18), thiényle, furyle ou pyrrolyle, imidazolyle, quinoléyle, pyrazolyle, pyridyle, pyrazinyle, pyrimidinyle ou pyridazinyle, les restes thiényle et furyle et l'hétérocycle étant non substitués ou substitués par 1 ou 2 substituants choisis dans le groupe constitué par les restes F, Cl, Br, CF₃, NO₂, CN, NH₂, OH, méthyle, éthyle, méthoxy, méthylamino, diméthylamino, éthylamino, diéthylamino, sulfamoyle, méthylsulfonyle et méthylsulfonylamino;
Y représente -O-, -CO-, -O-CO-, -S-, -SO-, -SO₂-, -SO₂-O-, -SO₂NR(12d)-, -NR(12d)- ou -CONR(12d)-, la liaison au cycle benzène se faisant toujours par l'intermédiaire de l'atome de gauche;
R(12d) est un atome d'hydrogène ou un reste méthyle ou éthyle;
s est 1, 2, 3, 4, 5 ou 6;
R(18) est un reste phényle substitué portant un ou deux substituants choisis dans le groupe constitué par les restes NH₂, N(méthyle)₂, OH, éthyle, -COOméthyle, -COOéthyle, -CONH₂, -CON(méthyle)₂; ou
R(18) représente un reste pyrrolyle, imidazolyle, quinoléyle, pyrazolyle, pyridyle, pyrazinyle, pyrimidinyle ou pyridazinyle portant un ou deux substituants choisis dans le groupe constitué par les restes F, CI, Br, CF₃, NO₂, CN, NH₂, OH, méthyle, éthyle, méthoxy, diméthylamino, sulfamoyle, méthylsulfonyle et méthylsulfonylamino; ou
R(18) représente un reste -OR(19), -NR(19)R(20), -CONR(19)R(20);
R(19) et R(20) représentent, indépendamment l'un de l'autre, un reste CₜH₂ₜ-R(21);
t est 0, 1, 2, 3, 4, 5 ou 6;
R(21) représente un atome d'hydrogène ou un reste CF₃, NR(22)R(23), -OR(24), phényle, thiényle, pyrrolyle, imidazolyle, quinoléyle, pyrazolyle, pyridyle, pyrazinyle, pyrimidinyle ou pyridazinyle, les restes phényle, thiényle et hétérocycliques étant non substitués ou substitués par 1 ou 2 substituants choisis dans le groupe constitué par les restes F, Cl, Br, CF₃, NO₂, CN, NH₂, OH, méthyle, éthyle, méthoxy, diméthylamino, sulfamoyle, méthylsulfonyle et méthylsulfonylamino;
R(22) et R(23) représentent, indépendamment l'un de l'autre, un atome d'hydrogène ou un reste alkyle de 1, 2 ou 3 atomes de carbone; ou
R(22) et R(23) forment ensemble une chaîne de 4 ou 5 groupes méthylène dont un groupe CH₂ peut être remplacé par -O-, -S-, -NH- ou -N(CH₃)-;
R(24) représente un atome d'hydrogène ou un reste alkyle de 1, 2 ou 3 atomes de carbone:
R(5), R(7) et R(8) représentent, indépendamment les uns des autres, un atome d'hydrogène ou un reste F, Cl, Br, alkyle de 1, 2, 3, 4 ou 5 atomes de carbone, CN, CF₃, NO₂, OR(12e);
R(12e) représente un reste alkyle de 1, 2, 3 ou 4 atomes de carbone;
R(9) représente un atome d'hydrogène ou un reste OH;
B représente un atome d'hydrogène, ou
R(9) et B forment ensemble une liaison;
et leurs sels physiologiquement acceptables.

3. Composés de formule I selon les revendications 1 à 2, dans lesquels:
R(1) et R(2) représentent, indépendamment l'un de l'autre, un atome d'hydrogène ou un reste CF₃ ou alkyle de 1 ou 2 atomes de carbone; ou
R(1) et R(2) forment ensemble une chaîne alkylène de 2, 3, 4 ou 5 atomes de carbone;
R(3) représente un groupe méthyle ou éthyle;
R(4) représente un groupe R(13)-CᵣH₂ᵣ dans lequel un groupe CH₂ du groupe CᵣH₂ᵣ peut être remplacé par -O-, -CO-O-, -O-CO-, -NR(14)- ou -CONR(14)-;
R(14) représente un atome d'hydrogène ou un reste alkyle de 1 ou 2 atomes de carbone;
R(13) représente un atome d'hydrogène ou un reste CF₃, -NR(15)R(16), - CONR(15)R(16), -OR(17), -COOR(17), phényle, pyrrolyle, imidazolyle, quinoléyle, pyrazolyle, pyridyle, pyrazinyle, pyrimidinyle ou pyridazinyle, le reste phényle et l'hétérocycle étant non substitués ou substitués par 1 ou 2 substituants choisis dans le groupe constitué par les restes F, CI, Br, CF₃, méthyle, méthoxy, sulfamoyle, méthylsulfonyle et méthylsulfonylamino;
R(15) et R(16) représentent, indépendamment l'un de l'autre, un atome d'hydrogène ou un reste alkyle de 1 ou 2 atomes de carbone; ou
R(15) et R(16) forment ensemble une chaîne de 4 ou 5 groupes méthylène dont un groupe CH₂ peut être remplacé par -O-, - NH- ou -N(CH₃)-;
R(17) représente un atome d'hydrogène ou un reste alkyle de 1 ou 2 atomes de carbone;
r est 1, 2, 3, 4, 5, 6 ou 7;
R(6) représente un reste -Y-CₛH₂ₛ-R(18), thiényle, furyle, pyrrolyle, imidazolyle, quinoléyle, pyrazolyle, pyridyle, pyrazinyle, pyrimidinyle ou pyridazinyle, les restes thiényle et furyle et l'hétérocycle étant non substitués ou substitués par 1 ou 2 substituants choisis dans le groupe constitué par les restes F, CI, Br, CF₃, méthyle, éthyle, méthoxy, méthylamino, diméthylamino, éthylamino, diéthylamino, sulfamoyle, méthylsulfonyle et méthylsulfonylamino;
Y représente -O- ou -CONR(12d)-, la liaison au cycle benzène se faisant par l'intermédiaire de l'atome de gauche;
R( 12d) est un atome d'hydrogène ou un reste méthyle ou éthyle;
s est 1, 2, 3, 4, 5 ou 6;
R(18) est un reste phényle substitué portant un ou deux substituants choisis dans le groupe constitué par les restes NH₂, N(méthyle)₂, OH, - COOméthyle, -COOéthyle, -CONH₂, -CON(méthyle)₂; ou
R(18) représente un reste pyrrolyle, imidazolyle, quinoléyle, pyrazolyle, pyridyle, pyrazinyle, pyrimidinyle ou pyridazinyle portant un ou deux substituants choisis dans le groupe constitué par les restes F, Cl, Br, CF₃, NO₂, CN, NH₂, OH, méthyle, éthyle, méthoxy, diméthylamino, sulfamoyle, méthylsulfonyle et méthylsulfonylamino; ou
R(18) représente un reste -OR(19) ou -CONR(19)R(20);
R(19) et R(20) représentent, indépendamment l'un de l'autre, un reste CₜH₂ₜ-R(21);
t est 0, 1, 2 ou 3;
R(21) représente un atome d'hydrogène ou un reste CF₃, NR(22)R(23), -OR(24);
R(22) et R(23) représentent, indépendamment l'un de l'autre, un atome d'hydrogène ou un reste alkyle de 1, 2 ou 3 atomes de carbone; ou
R(22) et R(23) forment ensemble une chaîne de 4 ou 5 groupes méthylène dont un groupe CH₂ peut être remplacé par -O-, -S-, -NH- ou -N(CH₃)-;
R(24) représente un atome d'hydrogène ou un reste alkyle de 1 ou 2 atomes de carbone:
R(5), R(7) et R(8) représentent un atome d'hydrogène;
R(9) représente un atome d'hydrogène ou un reste OH;
B représente un atome d'hydrogène, ou
R(9) et B forment ensemble une liaison;
et leurs sels physiologiquement acceptables.

4. Composés de formule I selon les revendications 1 à 3, dans lesquels:
R(1) et R(2) représentent un reste méthyle;
R(3) représente un reste méthyle ou éthyle;
R(4) représente un groupe R(13)-CᵣH₂ᵣ dans lequel un groupe CH₂ du groupe CᵣH₂ᵣ peut être remplacé par -O-;
R(13) représente un atome d'hydrogène ou un reste CF₃;
r est 1, 2, 3, 4, 5 ou 6;
R(6) représente un reste -Y-CₛH₂ₛ-R(18), thiényle, pyrrolyle, imidazolyle, quinoléyle, pyrazolyle, pyridyle, pyrazinyle, pyrimidinyle ou pyridazinyle, le reste thiényle et l'hétérocycle étant non substitués ou substitués par 1 ou 2 substituants choisis dans le groupe constitué par les restes F, Cl, CF₃, méthyle, méthoxy, diméthylamino, sulfamoyle, méthylsulfonyle et méthylsulfonylamino;
Y représente -O-;
s est 1, 2, 3, 4, 5 ou 6;
R(18) représente un reste pyrrolyle, imidazolyle, quinoléyle, pyrazolyle, pyridyle, pyrazinyle, pyrimidinyle ou pyridazinyle portant un ou deux substituants choisis dans le groupe constitué par les restes F, Cl, CF₃, NO₂, CN, OH, méthyle, méthoxy, diméthylamino, sulfamoyle, méthylsulfonyle et méthylsulfonylamino; ou
R(18) représente un reste -OR(19) ou -CONR(19)R(20);
R(19) et R(20) représentent, indépendamment l'un de l'autre, un reste CₜH₂ₜ-R(21);
t est 0, 1, 2 ou 3;
R(21) représente un atome d'hydrogène ou un reste CF₃, NR(22)R(23), -OR(24);
R(22) et R(23) représentent, indépendamment l'un de l'autre, un atome d'hydrogène ou un reste alkyle de 1 ou 2 atomes de carbone;
R(24) représente un atome d'hydrogène ou un reste alkyle de 1 ou 2 atomes de carbone:
R(5), R(7) et R(8) représentent un atome d'hydrogène;
R(9) représente un atome d'hydrogène;
B représente un atome d'hydrogène.

5. Composés de formule I selon l'une ou plusieurs des revendications 1 à 4 et leurs sels physiologiquement acceptables à utiliser comme médicaments.

6. Composition pharmaceutique contenant comme substance active une quantité efficace d'au moins un composé de formule I selon l'une ou plusieurs des revendications 1 à 4 et/ou un de leurs sels physiologiquement acceptables, avec des supports ou des additifs pharmaceutiquement acceptables et éventuellement une ou plusieurs autres substances actives pharmacologiques.

7. Utilisation d'un composé de formule I selon l'une ou plusieurs des revendications 1 à 4 et/ou d'un de leurs sels physiologiquement acceptables pour la préparation d'un médicament ayant une activité de blocage des canaux K⁺ pour la thérapie et la prophylaxie de maladies dues aux canaux K⁺.

8. Utilisation d'un composé de formule I selon l'une ou plusieurs des revendications 1 à 4 et/ou d'un de leurs sels physiologiquement acceptables pour la préparation d'un médicament destiné à inhiber la sécrétion d'acide gastrique.

9. Utilisation d'un composé de formule I selon l'une ou plusieurs des revendications 1 à 4 et/ou d'un de leurs sels physiologiquement acceptables pour la préparation d'un médicament destiné à la thérapie ou à la prophylaxie d'ulcères de l'estomac ou de la région intestinale.

10. Utilisation d'un composé de formule I selon l'une ou plusieurs des revendications 1 à 4 et/ou d'un de leurs sels physiologiquement acceptables pour la préparation d'un médicament destiné à la thérapie ou à la prophylaxie du reflux oesophagien.

11. Utilisation d'un composé de formule I selon l'une ou plusieurs des revendications 1 à 4 et/ou d'un de leurs sels physiologiquement acceptables pour la préparation d'un médicament destiné à la thérapie ou à la prophylaxie des maladies diarrhéiques.

12. Utilisation d'un composé de formule I selon l'une ou plusieurs des revendications 1 à 4 et/ou d'un de leurs sels physiologiquement acceptables pour la préparation d'un médicament destiné à la thérapie ou à la prophylaxie de tous les types d'arythmies, y compris des arythmies auriculaires, ventriculaires et supraventriculaires.

13. Utilisation d'un composé de formule I selon l'une ou plusieurs des revendications 1 à 4 et/ou d'un de leurs sels physiologiquement acceptables pour la préparation d'un médicament destiné à la thérapie ou à la prophylaxie des troubles du rythme cardiaque pouvant être éliminés par un prolongement du potentiel d'action.

14. Utilisation d'un composé de formule I selon l'une ou plusieurs des revendications 1 à 4 et/ou d'un de leurs sels physiologiquement acceptables pour la préparation d'un médicament destiné à la thérapie ou à la prophylaxie de la fibrillation auriculaire ou du flutter auriculaire.

15. Utilisation d'un composé de formule I selon l'une ou plusieurs des revendications 1 à 4 et/ou d'un de leurs sels physiologiquement acceptables pour la préparation d'un médicament destiné à la thérapie ou la prophylaxie d'arythmies de réentrée ou à empêcher la mort soudaine par arrêt cardiaque à la suite d'une fibrillation ventriculaire.

16. Utilisation d'un composé de formule I selon l'une ou plusieurs des revendications 1 à 4 et/ou d'un de leurs sels physiologiquement acceptables pour la préparation d'un médicament destiné à la thérapie de l'insuffisance cardiaque.

17. Utilisation d'un composé de formule I selon l'une ou plusieurs des revendications 1 à 4 et/ou d'un de leurs sels physiologiquement acceptables pour la préparation d'un médicament destiné à inhiber la sécrétion d'acide gastrique, à la thérapie ou à la prophylaxie d'ulcères de l'estomac ou de la région intestinale, du reflux oesophagien, des maladies diarrhéiques, à la thérapie ou à la prophylaxie d'arythmies, y compris des arythmies auriculaires, ventriculaires et supraventriculaires, de la fibrillation auriculaire et du flutter auriculaire et des arythmies de réentrée, ou à empêcher la mort soudaine par arrêt cardiaque à la suite d'une fibrillation ventriculaire.
